(19) Europäisches Patentamt
European Patent Office
Office européen des brevets

(11) Publication number: **0 318 680 B1**

# (12) EUROPEAN PATENT SPECIFICATION

(45) Date of publication of patent specification: **19.01.94**

(51) Int. Cl.5: **C07K 11/00**, C12P 21/04, //A61K37/02,C07K9/00, (C12P21/04,C12R1:045)

(21) Application number: **88116947.8**

(22) Date of filing: **13.10.88**

(54) **Novel antibiotic compounds named A/16686 Factors A'1, A'2 and A'3.**

(30) Priority: **27.11.87 GB 8727807**

(43) Date of publication of application:
**07.06.89 Bulletin 89/23**

(45) Publication of the grant of the patent:
**19.01.94 Bulletin 94/03**

(84) Designated Contracting States:
**AT BE CH DE ES FR GB GR IT LI LU NL SE**

(56) References cited:
**EP-A- 0 046 201**

**JOURNAL OF ANTIBIOTICS, vol. XXXVII, no. 4, 1984, pages 309-317, Tokyo, JP; B. CAVALLERI et al.: "A-16686, a new antibiotic from Actinoplanes; I. Fermentation, isolation and preliminary physico-chemical characteristics"**

(73) Proprietor: **GRUPPO LEPETIT S.p.A.**
**Via Roberto Lepetit, 8**
**I-20020 Lainate (MI)(IT)**

(72) Inventor: **Assi, Francesco**
**34, Via Corridoni**
**I-20063 Cernusco Sul Naviglio (Milano)(IT)**
Inventor: **Gastaldo, Luciano**
**7, Via San Martino**
**I-20010 Pogliano Milanese (Milano)(IT)**
Inventor: **Denaro, Maurizio**
**41, Viale Bligny**
**I-20136 Milano(IT)**
Inventor: **Ciabatti, Romeo**
**15-A, Via Brodolini**
**I-20026 Novate Milanese (Milano)(IT)**
Inventor: **Cavalleri, Bruno**
**25, Via Pierlombardo**
**I-20135 Milano(IT)**

(74) Representative: **Sgarbi, Renato et al**
**GRUPPO LEPETIT S.p.A.**
**Patent and Trademark Department**
**Via Roberto Lepetit, 34**
**I-21040 Gerenzano (Varese) (IT)**

EP 0 318 680 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention regards depsipeptidic compounds of following structure formula I

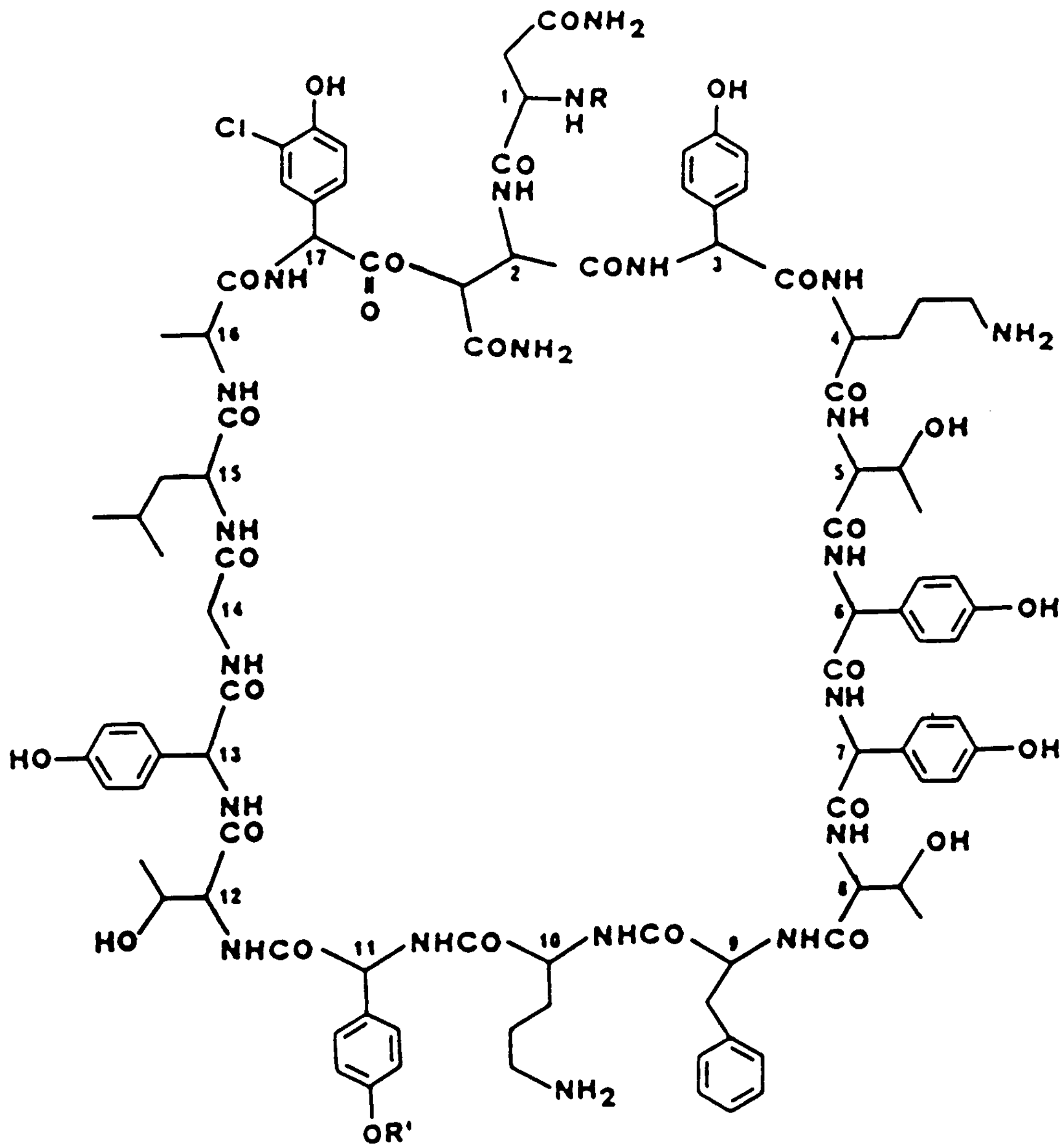

wherein:

R is:

-CO-CH = CH-CH = $CH-CH_2-CH_2-CH_3$,
-CO-CH = CH-CH = $CH-CH_2-CH(CH_3)_2$ or
-CO-CH = CH-CH = $CH-CH_2-CH_2-CH(CH_3)_2$

and R' is alpha-D-mannopyranosyl
and the acid addition salts thereof, the process for their preparation and their use as antibiotics. The above mentioned substances are correlated with antibiotic A/16686 and are produced by the same microorganism Actinoplanes sp. ATCC 33076 which produces the above mentioned antibiotic.

Antibiotic A/16686 is a substance active against gram-positive bacteria described in U.S. Patent 4,303,646 together with its manufacture process and pharmaceutical compositions containing it.

It was then found that three closely related components could be isolated from antibiotic A/16686 which were named factors A1, A2 and A3. Factor A2 (ramoplanin) is the component obtained in preponderant

amount and is the most relevant for the biological activity, while factors A1 and A3 are obtained in a minor amount. These substances as well as their preparation and uses are described in U.S. Patent 4,427,656.

A method for selectively enhancing the production of factors A2 and/or A3 of antibiotic A/16686 by adding appropriate precursors to an A/16686 producing culture is described in European Patent Application Publication No. 0259780.

The three compounds of this invention, which for the sake of brevity, are identified also as A/16686 factor A'1 (R = $-CO-CH=CH-CH=CH-CH_2-CH_2-CH_3$), factor A'2 (R = $-CO-CH=CH-CH=CH-CH_2-CH-(CH_3)_2$) and factor A'3 (R = $-CO-CH=CH-CH=CH-CH_2-CH_2-CH(CH_3)_2$), respectively, may be produced by fermentation of Actinoplanes sp. ATCC 33076, a strain which has been deposited with the permanent culture collection ATCC as described in U.S. Patent 4,303,646 and is now freely available and accepted under the Budapest Treaty as of January 31, 1981, or a producing mutant thereof (i.e., natural or artificial mutant capable of producing the same substances) under submerged aerobic conditions, in an aqueous nutrient medium containing assimilable sources of carbon and nitrogen and inorganic salts. To produce workable amounts (in this case, the term "workable amounts" is intended to mean that the amount of the invention compounds contained in the crude isolate from the fermentation broth is sufficient to permit their isolation with the usual separation and purification techniques in a quantity suitable for experimental purposes and practical utilization) of the three above mentioned compounds in the crude fermentation product, the fermentation media must contain appropriate sources of the essential elements mentioned above.

Preferred carbon sources are sugars such as dextrose, fructose, maltose, sucrose and the like, polyols such as glycerol and the like, starch and modified starches such as dextrin, with sucrose, glycerol and dextrin being the most preferred. However, it appears that when the concentration of dextrose in the nutrient culture medium is moderate or low, this has a positive effect on the formation of the compounds of formula I as it can be inferred from their larger proportion in the crude isolate from the fermentation broth.

Preferred nitrogen sources are soybean meal, peptone, tryptone, malt extract, yeast extract, aminoacids and the like with soybean meal and malt extract being the most preferred.

Among the inorganic salts which are usually incorporated in the culture media are the customary soluble salts capable of yielding sodium, potassium, iron, zinc, cobalt, manganese, magnesium, calcium, ammonium, chloride, iodide, carbonate, sulfate, phosphate, nitrate ions and the like.

Addition of appropriate precursors during the fermentation according to the procedure described in European Patent Application Publication No. 0259780 may be useful to selectively increase the ratio of one of the new antibiotics of formula I over the others. For instance, addition of from 0.2 g/l to 5 g/l of leucine (or its salts) increases the ratio of factor A'2 relative to factors A'1 and A'3 while addition of from 0.2 g/l to 5 g/l of valine (or its salts) selectively increases the relative ratio of factor A'3. Isovaleric acid has the same effect as leucine while isobutyric acid has the same effect as valine.

Ordinarily, the antibiotic-producing strain is pre-cultured in a shake flask, then the culture is used to inoculate jar fermentors for production of substantial quantities of the antibiotic substances. The medium used for the pre-culture can be the same as that employed for larger fermentations, but other media can also be employed. The producing-strain can be grown at temperatures between 20 and 40 °C, preferably between 24 and 35 °C, most preferably between 28 and 32 °C for a period of time (generally varying from 30 to 180 hours) during which an increase of antibiotic activity is observed.

During fermentation, the antibiotic production can be monitored by testing broth or mycelial extract samples for antibiotic activity, for instance, by bioassays or TLC or HPLC procedures.

Sensitive organisms to the antibiotics of this invention such as Bacillus subtilis or Bacillus pumilus can be used as test organisms. The bioassay is conveniently performed by the agar diffusion method on agar plates.
Maximum production of antibiotic activity generally occurs between the third and the fifth day after inoculation.

The antibiotics produced during fermentation of the strain Actinoplanes sp. ATCC 33076 are found mainly in the mycelial mass. Therefore, the antibiotics of this invention are suitably recovered by separating the mycelium from the fermentation broth, extracting the mycelial mass with an appropriate solvent system, isolating the crude fermentation product from said extract, separating and purifying the above antibiotic substances from the isolated crude fermentation product. A preferred method of recovering the A/16686 factors A'1, A'2 and A'3 antibiotics comprises extraction of the wet mycelium after separation from the fermentation broth by filtration at a pH between 4.5 and 5.5.

Extraction of the mycelial mass is best accomplished with water miscible organic solvents such as lower alkanols, acetone and their mixtures at a pH between 1.5 and 2.5.
A crude fermentation product (the "crude"), which is a mixture of A/16686 antibiotics, i.e. factors A1, A2 and

A3 (U.S.Patent 4,427,656) admixed with compounds of this invention, i.e., factor A'1, A'2 and A'3 together with impurities, side products, salts, filter aids and materials deriving from the fermentation broth components, is recovered from the extracting solvent(s) by routine procedures.

The isolation of the antibiotic substances of this invention from the crude fermentation product, their separation and purification are conducted according to known per se techniques which include extraction with solvents, precipitation by adding non-solvents or by changing the pH of the solution, partition chromatography, reverse-phase partition chromatography, ion-exchange chromatography, affinity chromatography, HPLC techniques and the like.

According to a typical method of recovering the "crude", the mycelial extract in a mixture of water and water miscible organic solvents at acidic pH, which contains the above antibiotics in the form of acid addition salt, is usually extracted with an organic solvent poorly miscible with water and having a high dissolving power for lipophilic compounds (e.g., ethyl acetate, ethyl ether, n-hexane) and then the aqueous layer is concentrated under vacuum. The concentrated solution is then brought to pH 7 by addition of diluted alkali or ammonia and then filtered or centrifuged to recover the solid. Filter aids can be also added to the mixture to favor the recovery of the solid product.

The wet "crude" cake obtained can be further elaborated for isolation and purification of the compounds of formula I above. If a filter aid has been added before filtration it may be necessary to submit the "crude" cake to a further extraction with a mixture of water and a water miscible organic solvent at acidic pH like to the one applied for the extraction of the mycelial cake.

Re-precipitation of the "crude" from this extract is carried out by decreasing the solubility of the acid addition salts of the invention products in the extract by adding thereto a water miscible organic solvent wherein said acid addition salts are poorly soluble. Acetone and isopropanol are suitable organic solvents for said purpose.

The wet crude isolate is then further purified for eliminating therefrom most of the non-A/16686 antibiotic products. Thus, sludging the crude wet cake with a diluted (5 to 10% w/v)) solution of a strong mineral acid followed by centrifugation is useful for eliminating some of the impurities. The wet cake thus obtained may be further purified by re-dissolving in a mixture of water and water soluble organic solvent of the same type as those employed for the extraction of the mycelial cake at a pH between 1.5 and 2.5. During this operation decolorizing agents can be added to the aqueous solution. Precipitation of the purified fermentation product containing the compound of formula I from the acidic solution may be carried out according to the procedure described above. Such purified preparation of the fermentation product is essentially consisting of a mixture of the compounds of this invention together with one or more other similar products, i.e., the factors A1, A2 and A3 of antibiotic A/16686. Isolation of the compounds of this invention from the above described mixture is carried out by the usual separation techniques as mentioned above. Preparative HPLC is particularly useful for both separation and purification purposes when the fermentation products contain considerable amount of antibiotic A/16686 factors A1, A2 and A3. The preparative HPLC operations are usually conducted under conditions which are common to the separation and purification of the A/16686 antibiotic. Examples of said separation and purification operations can be found, for instance, in U.S. Patent 4,427,656 where a C-18 alkyl silanized silicagel column and an eluent mixture of aqueous ammonium formate and acetonitrile are employed.

During the preparative HPLC the eluted liquids from each injection are checked by analytical HPLC and those fractions enriched in A/16686 factors A'1, A'2 and A'3, respectively, are separated.

The fractions enriched in each of the above compounds are combined and concentrated to dryness under vacuum. The respective solid residues are re-submitted to preparative HPLC under the same conditions as before. The solid products resulting from concentration of the eluted solutions are dissolved in diluted mineral acids and freeze-dried to yield the respective pure products under the form of mineral acid addition salts.

An alternative method for producing the compounds of this invention consists in contacting a substrate containing antibiotic A/16686 factor A1, A2 or A3 or a mixture thereof with the mycelium of Actinoplanes sp. ATCC 33076 or a natural or artificial mutant thereof capable of producing A/16686 antibiotics thus promoting the biotransformation into factors A'1, A'2 and A'3. According to this procedure, the single factors A1, A2 and A3 or a mixture thereof, such as, for instance, the complex resulting from a fermentation operation, are allowed to contact the mycelium for a period of time ranging from 50 to 200 hours at a temperature between 28 and 35°C, preferably between 29 and 33°C. This operation may be sequential to the fermentation of Actinoplanes sp. ATCC 33076 (or a producing mutant thereof) for producing the A/16686 antibiotics and therefore may consist in a prolonged contact of the fermentation product with the mycelium. When the fermentation operations have been carried out according to the conditions described above, the fermentation product already contains some amounts of factors A'1, A'2 and A'3 and, therefore, a prolonged

contact with the mycelium actually results in an enrichment of the ratios of said compounds in the fermentation batch.

According to a practical embodiment of this method, after the fermentation cycle is complete, i.e., when analytical tests show that the antibiotic activity in the fermentation broth is no longer increasing, inlet of oxygen (or air) into the fermentors is stopped and the fermentation broth is kept with stirring at a temperature between 29 and 33°C for an additional period of time varying from 50 to 200 hours before harvesting under analytical HPLC control.

A preferred mode to carry out the biotransformation of the factors A1, A2 and A3 into the corresponding factors A'1, A'2 and A'3 consists in contacting the first three substances mentioned above (or a mixture thereof, including the A/16686 complex as resulting from the recovery operations from the fermentation batch, as described, for instance, in U.S. Patent 4,303,646) with the isolated mycelium of Actinoplanes sp. ATCC 33076 or a producing natural or artificial mutant thereof. Mycelium grown in a suitable culture medium is isolated from the fermentation broth (e.g. by centrifugation) and then added to a solution of factor A1, A2 or A3 or a mixture thereof in water or in a mixture of water and one or more organic solvents mixable with water (e.g. lower alkanols or acetone) with stirring. The temperature of the reaction mixture is maintained within the range indicated above while the pH may be kept steady around 7 by buffering the solution with a suitable buffer (e.g. a phosphate buffer). The biological transformation is usually followed by analytical HPLC. Experimental tests show that an A/16686 antibiotic crude mixture originally containing respectively about 14 percent of factor A1, about 64 percent of factor A2 and about 12 percent of factor A3 after about 140 hours of contact with the isolated mycelium of Actinoplanes sp. ATCC 33076 under the above conditions is transformed into a mixture containing respectively about 10% factor A'1, about 38% factor A'2 and about 5% factor A'3 with a concomitant decrease of the content of factors A1, A2 and A3 to 10%, 28% and 9% (HPLC areas), respectively. Analogously, a substrate consisting of an almost pure sample of factor A2 (HPLC titre: 89%) after 180 hours is converted into a mixture containing about 60% factor A'2 with the content of factor A2 decreasing to about 33% (HPLC areas).

The products resulting from the above described biotransformation are recovered from the reaction mixture (or from the fermentation broth, in case the biological transformation is directly operated by prolonging the contact with the mycelium in the fermentation batch) by acidifying the mycelial suspension to a pH between 1.5 and 2.5 and then adding thereto a water soluble organic solvent such as a lower alkanol or acetone, if this is not already present in a sufficient amount, to help extraction of the reaction products from the mycelial mass. After separation of the mycelium by filtration or centrifugation, the solution is elaborated in the same way as described above for isolating and purifying the compounds of this invention.

When the biological transformation is directly operated on the fermentation broth as mentioned above, the recovery of the reaction products can be carried out exactly as described before, for the recovery of the fermentation products.

Antibiotic A/16686 factors A'1, A'2 and A'3 are submitted to sugar content determination (acid hydrolysis), acid/base titration, amino acid analysis (for quantity and sequence), IR, UV, NMR spectrometry and Fast Atom Bombardment Mass Spectrometry (FAB-MS). The data resulting from these analytical tests confirm the assigned structures.

As shown in formula I the antibiotic substances of this invention possess basic functions which can form acid addition salts according to conventional procedures.

Representative and suitable acid addition salts of the compounds of formula I include those salts formed by standard reactions with both organic and inorganic acids such as, for example, hydrochloric, hydrobromic, sulfuric, phosphoric, acetic, trifluoroacetic, trichloroacetic, succinic, citric, ascorbic, lactic, maleic, fumaric, palmitic, cholic, pamoic, mucic, glutamic, camphoric, glutaric, glycolic, phthalic, tartaric, lauric, stearic, salicylic, methanesulfonic, benzenesulfonic, sorbic, picric, benzoic, cinnamic acid and the like.

The transformation of the free amino or non-salt compounds of the invention into the corresponding addition salts, and the reverse, i.e., the transformation of an addition salt of a compound of the invention into the non-salt form, are within the ordinary technical skill and are encompassed by the present invention.

For instance, a compound of the invention can be transformed into the corresponding acid addition-salt by dissolving the non-salt form in an aqueous solvent and adding a slight molar excess of the selected acid. The resulting solution or suspension is then lyophilized to recover the desired salt.

In case the final salt is insoluble in a solvent where the non-salt form is soluble it is recovered by filtration from the organic solution of the non-salt form after addition of the stoichiometric amount or a slight molar excess of the selected acid.

The non-salt form can be prepared from a corresponding acid salt dissolved in an aqueous solvent which is then neutralized to set free the non-salt form.

When following the neutralization desalting is necessary, a common desalting procedure may be employed.

For example, column chromatography on silanized silica gel, non-functionalized polystyrene, acrylic and controlled pore polydextrane resins (such as Sephadex® LH 20) or activated carbon may be conveniently used. After eluting the undesired salts with an aqueous solution, the desired product is eluted by means of a linear gradient or a step-gradient of a mixture of water and a polar or apolar organic solvent, such as acetonitrile:water from 50:50 to about 100% acetonitrile.

As it is known in the art, the salt formation either with pharmaceutically acceptable acids or non-pharmaceutically acceptable acids may be used as a convenient purification technique. After formation and isolation, the salt form of an antibiotic of formula I above can be transformed into the corresponding non-salt or into a pharmaceutically acceptable salt.

Antibiotic A/16686 factors A'1, A'2 and A'3 are particularly active against gram-positive microorganisms. The microbiological activity spectrum of antibiotic A/16686 factor A'2 is reported in the following Table:

TABLE I

| In vitro activity of A/16686 factor A'2 | |
|---|---|
| Strain | MIC (mcg/ml) |
| Staphylococcus aureus Tour | 1 |
| Staphylococcus aureus Tour [a)] | 2 |
| Staphylococcus aureus Tour [b)] | 1 |
| Staphylococcus epidermidis ATCC 12228 | 0.25 |
| Staphylococcus haemolyticus L 602 [c)] | 1 |
| Streptococcus pyogenes C203 | 0.008 |
| Streptococcus pneumoniae UC41 | 0.008 |
| Streptococcus faecalis ATCC 7080 | 0.5 |
| Streptococcus mitis L 796 [c)] | 0.032 |
| Clostridium perfringens ISS 30543 | 2 |
| Clostridium difficile ATCC 9689 | 1 |
| Propionibacterium acnes ATCC 6919 | 0.5 |
| Propionibacterium acnes ATCC 6922 | 0.125 |
| Propionibacterium acnes L 1557 [c)] | 0.125 |
| Propionibacterium acnes L 1559 [c)] | 0.125 |
| Propionibacterium acnes L 1563 [c)] | 0.125 |
| Propionibacterium acnes L 1565 [c)] | 0.125 |

[a)] Inoculum $10^6$ cfu/ml
[b)] 30% bovine serum added
[c)] Clinical isolates

Minimal Inhibitory Concentration (MIC) is determined by either the broth or the agar serial two-fold dilution method. Culture media and growth conditions:
Iso-Sensitest broth (Oxoid), for staphylococci and Streptococcus faecalis; Todd-Hewitt broth (Difco), for other streptococcal species; Wilkins-Chalgren agar for anaerobic bacteria (T.D. Wilkins, S. Chalgren: Antimicrob. Agents Chemother. 10, 926 (1976)); the final inoculum is of about $10^4$ colony-forming units/ml or spot. MIC is read as the lowest concentration which shows no visible growth after 18-24 hours incubation at 37°C; for anaerobs the incubation is at 37°C for 48 hours in anaerobic atmosphere ($N_2$:$CO_2$:$H_2$, 80:10:10). The acute toxicity of factor A'2 is determined in CD1 mice (Charles River) of both sexes weighing 18-22 g by a single i.v. injection of the product, solubilized in sterile saline. Infusion rate is 0.15 ml/second. Dose levels are 75, 100, 125 and 150 mg/kg. The $LD_{50}$ value calculated is 103 mg/kg.
The other two products show biological activities comparable with that of antibiotic A/16686 factor A'2.

The antibiotic compounds of this invention are useful for preparing medicaments against infections primarily due to gram-positive widely diffused bacteria. In particular, the compounds of this invention are useful for topical treatment of wound infections and acne.

For use as medicaments the compounds of this invention can be administered by different routes either in the form of free compounds or in the form of their addition salts with pharmaceutically acceptable acids, this latter form being preferred. The topical route is usually the most suitable way to administer the compounds of this invention.

For the medical uses the compounds of this invention are incorporated into pharmaceutical dosage forms suitable for oral, topical or parenteral administration such as tablets, capsules, lozenges, gelules, granules, powders, ointments, gels, liquid solutions, creams, solutions for injections, suspensions and the like. For instances, the formulations of said dosage forms can be carried out according to the general teaching of Remington's Pharmaceutical Sciences 17th Edition, 1985 Merck Publishing Company, Easton Pennsylvania.

The dosage unit may contain from 0.01 to 99 percent preferably from 0.5 to 80 percent of active ingredient. The daily dosage may depend on several factors such as body weight, the infecting microorganism, the severity of the infection, the age of the patient, the period and the way of administration. In general, the compounds of this invention are effective at a daily dosage ranging from about 2 mg to about 100 mg per kilogram of body weight, optionally divided into one or more administrations per day. Obviously, these dosages are only indicative and the most appropriate dosage can be adjusted in the specific cases and applications by relying on biological testings useful for determining the amount of active compound required to produce the desired effect.

The following Examples have the purpose to illustrate the invention but should not be construed as a limitation of its scope.

## EXAMPLES

### Example 1 - Fermentation of Actinoplanes sp. ATCC 33076 and recovery of the crude fermentation product

#### 1.1 Fermentation of Actinoplanes sp. ATCC 33076

A lyophilized vial of master stock is used to inoculate two 18 cm oat meal agar slants. After incubation for one week at 29 °C, the mycelium of one slant is inoculated into a baffled flask of 500 ml containing 100 ml vegetative medium (1), which is incubated at 29 °C with rotary agitation (200 r.p.m.).

After 48 hours the grown culture of one flask with pH = 7.0 is transferred to a 7-liter vessel with 4 liters of vegetative medium(1) (900 r.p.m.; 28 °C; 0.5 v/v/m of air). After 48 hours, 25 ml of the tank culture, having pH = 7.1, are dispensed into 50 ml ampoules. The ampoules are then frozen in a slurry of acetone and dry ice and stored at -74 °C.

The frozen mycelium of 10 ampoules is inoculated in 10 baffled flasks (2000/400 ml) of vegetative medium (1). After 51 hours of incubation at 29 °C with rotary agitation (150 r.p.m) the flasks have an average pH of 6.8. Subsequently, all 10 flasks are inoculated into a 10,500 l fermentor filled with 4000 l of vegetative medium(1) sterilized in batch at 120 °C for 20 minutes.

After 68 hours at 29 °C the preculture, which has a pH of 6.8, is used to inoculate two 27,500 l fermentors filled with 20,000 l each of fermentation medium (2) sterilizedin batch at 120 °C for 20 minutes. The fermentation temperature is kept constant at 29 °C and the fermentation course is followed by analytical HPLC and stopped after 65 hours.

| (1): Composition of vegetative medium | |
|---|---|
| Soybean meal | 13 g/l |
| Dextrose | 12 g/l |
| Dextrin | 13 g/l |
| Calcium carbonate | 4 g/l |

| (2): Composition of fermentation medium | |
|---|---|
| Soybean meal | 30 g/l |
| Glycerol | 20 g/l |
| Dextrose | 4 g/l |
| Dextrin | 4 g/l |
| Crude malt extract | 20 g/l |
| Sucrose | 20 g/l |
| Calcium carbonate | 6 g/l |

1.2 Recovery of the crude fermentation product

The harvest broth from the two fermentors is cooled at 10 °C and brought to pH 5 with 20% (w/v) HCl and filtered on a rotary filter.

The wet mycelium is separated and extracted with 12,500 l of acetone in three times by stirring the suspension at pH 2 (with 30% HCl) and centrifuging. The hydroacetonic extract is divided in four portions and extracted with a total volume of 12,600 l of ethyl acetate. The aqueous phase is concentrated under vacuum at 18 °C (internal temperature) to 7,000 l.

To the concentrated solution cooled at 4 °C, 150 l of 15% $NH_4OH$ is slowly added until pH 7, in the presence of 210 1 of $CH_2Cl_2$. After addition of 55 kg of filter aid the suspension is filtered on a rotary filter. The wet cake is re-extracted three times by using every time a mixture of 230 l of acetone and 44 l of water at pH 2 (20% HCl) with slurry at room temperature.

After washing the solid with a further portion of 110 l of a mixture acetone:water 6:4 at pH 2, the three solutions and washings are combined and 2,600 l of acetone is added thereto with stirring at room temperature. After standing overnight, the solid precipitate is centrifuged, washed with 240 l of acetone and dried at room temperature under vacuum yielding 54 kg of crude fermentation product having a 28% total A/16686 antibiotic HPLC titre.

Example 2 - Isolation and purification of the antibiotic A/16686 factors A'1, A'2 and A'3

2.1 Purified mixture of A/16686 antibiotics

A portion of 13 kg of the crude fermentation product obtained according to Example 1 is sludged with 80 l of 5% (w/v) HCl at about 15 °C by vigorous stirring for 3 hours. The suspension is centrifuged and the solid is washed with 24 l of 5% HCl. The wet cake is extracted with a mixture of 80 l of acetone and 33 l of water in the presence of 10.5 kg of decolorizing diatomaceous earth (Tonsil Optimum NFF, Süd-Chemie A.G., München) and 2.1 kg of charcoal (Darco G60) at 15 °C. The suspension is centrifuged and the solid is washed with 30 l of a mixture acetone:water 6:4. The extracts are combined (140 l in total) and 350 l of acetone are added slowly thereto with stirring. After standing overnight the mixture is filtered on a series of Buchner filters. The resulting cake is washed with 10 l of a mixture acetone:water 9:1 and then with 4 l of acetone. The solid product remaining on the filter is dried under vacuum for 72 hours at room temperature to yield 1.86 kg of a purified preparation (89% HPLC titre) of A/16686 antibiotics which are used for further elaborations to isolate the compounds of this invention.

2.2 Isolation and purification of antibiotic A/16686 factors A'1, A'2 and A'3

27 Grams of the preparation obtained according to paragraph 2.1 is submitted to preparative HPLC by using the following apparatus and conditions for each portion of 300 mg dissolved in 5 ml of water containing 10% (v/v) of $CH_3CN$.

Instrument: The apparatus is set up by assembling a Waters mod. 590 pump, a Waters Lambda-Max mod. 481LC detector set at 285 nm, and a Rheodyne injector equipped with a 5 ml loop.

Column: LiChrosorb RP-18, 10 micron, 250 mm x 50 mm (Merck)

Mobile phase: 0.05 M $HCOONH_4$:$CH_3CN$ (64:36)

Flow rate: 30 ml/min

The operations are monitored by analytical HPLC (see Example 5, paragraph 5.1). The groups of fractions enriched respectively in factor A'1, factor A'2 and factor A'3 are separated and each group of fractions is combined to form three solutions which are worked in the same manner as follows. The solution

is concentrated to dryness under vacuum and the residue is purified again by preparative HPLC. The fractions containing the pure component are pooled and concentrated under vacuum. The solid residue is taken up twice with ethanol and collected by filtration. The dry solid is dissolved in 0.1N HCl and freeze-dried to yield the hydrochloride of the pure component which is submitted to analysis and physico-chemical characterization.

From the above mentioned sample the following amounts of pure hydrochlorides of factors A'1, A'2 and A'3 are respectively obtained: 150 mg, 600 mg and 30 mg.

Example 3 - Biological transformation of antibiotic A/16686 factor A2 into factor A'2

A sample (600 mg) of antibiotic A/16686 factor A2 (HPLC titre 89%) prepared by following the procedure described in U.S. Patent 4,427,656 is dissolved in 200 ml of water (in a 1000 ml flask). To this solution is added the mycelium of Actinoplanes sp. ATCC 33076 obtained by centrifugation of 400 ml of a culture grown in vegetative medium in a 7 liter vessel (as described under Example 1, paragraph 1.1 for preparing the inoculation mycelium).
The mixture is stirred at 200 r.p.m. at 32 °C and the biological transformation is followed by analytical HPLC (see under Example 5, paragraph 5.1).

The HPLC analysis of the mycelial suspension is carried out every 2 hours. The analytical pattern shows that the mycelial suspension which originally contains factor A2 in a concentration of 2053 micrograms/ml after 180 hours contains factor A2 and factor A'2 in concentrations of 282 micrograms/ml (33%) and 513 micrograms/ml (60%), respectively.

The suspension is worked up in the same manner as the fermentation broth in Example 1, first part of paragraph 1.1, to obtain a hydroacetonic extract of the mixture of antibiotic A/16686 factor A2 and factor A'2 which is then precipitated from said extract by adding acetone in a proportion of about 3 volumes for each volume of hydroacetonic extract.

The solid precipitate is then separated by filtration and worked up as in the last part of Example 2, paragraph 2.1, to yield a purified mixture of antibiotic A/16686 factor A2 and factor A'2 with an HPLC titre of about 33% and about 60%, respectively. Isolation and purification of factor A'2 from said mixture are carried out according to paragraph 2.2 of Example 2 yielding 55 mg of pure antibiotic A/16686 factor A'2 hydrochloride.

Example 4 - Biological transformation of a mixture of antibiotic A/16686 factors A1, A2 and A3 into a mixture containing factors A'1, A'2 and A'3

A sample of 600 mg of antibiotic A/16686 complex containing factor A1 13.9%, factor A2 64.3%, factor A3 12.3% (analytical HPLC) is submitted to the same procedure as described in Example 3. The reaction is stopped after 140 hours during which the reaction is monitored by HPLC. The analytical pattern of the mycelial suspension shows that the concentration of factors A1, A2 and A3 which at the origin was 313 micrograms/ml, 1434 micrograms/ml and 227 micrograms/ml, respectively, after 140 hours has decreased to 96 micrograms/ml (10%), 273 micrograms/ml (29.4%) and 85 micrograms/ml (8.9%), respectively, while the concentration of factors A'1, A'2 and A'3 is 99 micrograms/ml (10.3 %), 364 micrograms/ml (37.9%) and 45 micrograms/ml (4.7%), respectively.

The suspension is worked out as described in Example 3 above and to yield a purified mixture of A/16686 antibiotics with the following HPLC titre: factor A1 (10%), factor A2 (28%), factor A3 (9%), factor A'1 (10%), factor A'2 (38%), factor A'3 (5%). From this preparation factors A'1, A'2 and A'3 are isolated in the form of the respective hydrochlorides by following the same procedure described under Example 2, paragraph 2.2.

Example 5 - Analytical assays and physico-chemical characterization

5.1 Analytical HPLC

Apparatus: Hewlett-Packard liquid chromatograph, mod. 1084 B equipped with a UV detector set at 254 nm.
Column: Erbasil C-18, 5 micron, 150 mm x 4.6 mm (Carlo Erba)
Mobile phase:
A) 0.05 M $NaH_2PO_4$ pH 4;
B) 0.05 M $NaH_2PO_4$ pH 4:$CH_3CN$ 73:23
Flow rate: 0.5 ml/min

| Gradient profile: | min<br>% B | 0<br>45 | 10<br>45 | 40<br>65 |
|---|---|---|---|---|

Injection volume: 20 microliters of a solution of the substance to be examined at about 0.5 mg/ml in water
Under these conditions the respective retention times ($t_R$) are as follows:

| | $t_R$ (minutes) |
|---|---|
| factor A1 | 15.14 |
| factor A'1 | 17.81 |
| factor A2 | 20.30 |
| factor A'2 | 22.70 |
| factor A3 | 24.85 |
| factor A'3 | 27.24 |

When the assay is performed on the fermentation broth, the sample to be analyzed is prepared as follows:
two milliliters of the fermentation broth are drawn and 40 microliters of 6N HCl are added thereto to bring the pH value between 1.8 and 2.0. Two milliliters of acetone are added to the mixture which is stirred for ten minutes and then centrifuged for five minutes at 3000 r.p.m. The mycelium is separated and a portion of two milliliters of the solution is extracted with an equal volume of ethyl acetate. After separation of the two phases the aqueous layer is separated (about 1.2 ml) and used for HPLC determination.

5.2 Sugar analysis

Sugar determination is made after acid hydrolysis (2N $H_2SO_4$, 100 °C, 2 hours) as described by B. Cavalleri et al. in The Journal of Antibiotics, Vol. 37, No. 4, pp 309-317, 1984. The presence of one D-mannose unit for each molecule of the three factors A'1, A'2 and A'3 is observed.

5.3 Amino acid analysis and $^1$H-NMR spectra

The acid hydrolysis is performed on all three compounds with 6N HCl at 105 °C for 20 hours in a sealed tube. The mixture of amino acids is separated by column chromatography on a strongly acidic sulfonic divinylbenzene resin (Dowex 50 W) by eluting with aqueous HCl of increasing concentrations from 0.5N to 2N.

The amino acids are identified by comparison with authentic samples on the basis of $^1$H-NMR and GC-MS. The amino acid ratio and their sequence in the intact molecules are determined by NMR experiments.

All three compounds show the same amino acids compositions and sequence.
The following Table II shows the type and number of amino acid residues in each of the three factors A'1, A'2 and A'3.

Table II

| Amino acid | Number of units |
|---|---|
| Threo-beta-hydroxyaspartic acid | 1 |
| Aspartic acid | 1 |
| Allo-threonine | 3 |
| Glycine | 1 |
| Alanine | 1 |
| 4-Hydroxyphenylglycine | 5 |
| Leucine | 1 |
| Phenylalanine | 1 |
| 3-Chloro-4-hydroxyphenylglycine | 1 |
| Ornithine | 2 |

The equivalents of ammonia per mole of each factor are titrated on the respective acid hydrolysis mixture by means of an amino acid automatic analyzer providing evidence of two primary amide groups.

Furthermore, the total number of amidic nitrogen atoms (19) resulting from $^{15}N$ NMR experiments exceeds by two the number of nitrogen atoms involved in the peptide bonds according to the number of amino acids in the molecule (Table II). Titration of the factors does not show any presence of free carboxylic groups. These considerations support that the two primary amide groups are on the aspartic and threo-beta-hydroxyaspartic acid units, respectively.

The NMR spectra are recorded in the temperature range from 25 to 60 °C on a Bruker AM 250 spectrometer equipped with an Aspect 3000 computer.

Standard pulse sequence and standard software are used for the 2D-NMR spectra, with slight modifications to suppress the water peak during the measurement.

The following 2D techniques are used: COSY, Relayed COSY, (W.J. Chazin, D.P. Goldenberg, T.E. Creighton, K. Wüthrich: Eur.J. Biochem., 152, 429-437 (1985)), COSY with enhancement of long range couplings (A. Bax, R. Freeman: J. Magn. Reson., 44, 542-561 (1981)), NOESY (S. Macura, K. Wüthrich, R.R. Ernst: J. Magn. Reson. 47; 351-357 (1982)) and COLOC (H.Kessler, C.Griesinger, J. Zarbock, H.R. Loosli: J. Magn. Reson. 57, 331-336 (1984)).

The following Table III shows the $^1H$-NMR chemical shifts (delta, ppm) of the amino acids of the amino acidic moiety of antibiotic A/16686 factor A'2 in
$H_2O$ : DMSO, 4:1 at pH 4.6, temp. 40 °C, internal standard TMS (delta = 0.00 ppm)

TABLE III

| | Amino acid | HN | $HC_{alpha}$ | $HC_{beta}$ | others |
|---|---|---|---|---|---|
| 1 | Aspartic acid | 8.09 | 4.76 | 2.17, 1.62 | - |
| 2 | Beta-hydroxyaspartic acid | 8.41 | 5.49 | 5.69 | - |
| 3 | 4-Hydroxyphenylglycine | 9.84 | 6.21 | - | Phenyl 7.64(b,f), 7.02(c,e) |
| 4 | Ornithine | 9.20 | 4.21 | 2.04 | - |
| 5 | Threonine | 7.56 | 4.36 | 4.03 | $HC_{gamma}$ 1.04 |
| 6 | 4-Hydroxyphenylglycine | 8.91 | 5.44 | - | Phenyl 6.67(b,f), 6.45(c,e) |
| 7 | 4-Hydroxyphenylglycine | 8.92 | 6.78 | - | Phenyl 6.82(b,f), 6.36(c,e) |
| 8 | Threonine | 8.24 | 3.71 | 3.92 | $HC_{gamma}$ 0.82 |
| 9 | Phenylalanine | 7.74 | 4.97 | 2.04 | Phenyl 7.22(b,f), 6.92(c,d,e,) |

TABLE III (continued)

| | Aminoacid | HN | $HC_{alpha}$ | $HC_{beta}$ | others |
|---|---|---|---|---|---|
| 10 | Ornithine | 7.59 | 4.20 | 2.18, 1.89 | $HC_{gamma}$ 1.67, $HC_{delta}$ 3.02 |
| 11 | 4-Hydroxyphenylglycine | 9.23 | 6.94 | - | Phenyl 7.34(b,f), 6.95(c,e) |
| 12 | Threonine | 9.06 | 4.72 | 3.94 | $HC_{gamma}$ 0.95 |
| 13 | 4-Hydroxyphenylglycine | 8.78 | 6.09 | - | Phenyl 7.08(b,f), 6.70(c,e) |
| 14 | Glycine | 7.89 | 3.76,2.99 | - | - |
| 15 | Leucine | 8.36 | 4.26 | 1.47 | $HC_{gamma}$ 1.47, $HC_{delta}$ 0.74 |
| 16 | Alanine | 9.32 | 4.32 | 1.41 | - |
| 17 | 3-Chloro-4-hydroxyphenyl-glycine | 7.87 | 4.91 | - | Phenyl 6.83(f), 6.51(b), 6.36(e) |

Factors A'1 and A'3 show the same pattern.

Figure 1 reports the $^1$H-NMR spectrum of factor A'2.

$^1$H-NMR spectra of factors A'1 and A'3 show the same pattern as that of factor A'2, apart from the signals attributed to the fatty acid chains attached to the aspartic moiety.

5.4 Fatty acid moieties

The following Table IV summarizes the $^1$H-NMR chemical shifts (delta, ppm) assignments of the fatty acid moiety of each of the three compounds in $H_2O$:DMSO, 4:1 at pH 4.6, temperature 40 °C, internal standard TMS (delta = 0.00 ppm).

TABLE IV

| Factor | HC-2 | HC-3 | HC-4 | HC-5 | HC-6 | Others |
|---|---|---|---|---|---|---|
| A'1 | 5.52 | 6.25 | 7.26 | 6.12 | 2.00 | 1.67 (2H) HC-7; 0.85 (3H) HC-8 |
| A'2 | 5.52 | 6.25 | 7.26 | 6.12 | 2.00 | 1.67 (1H) HC-7; 0.81, 0.82 (6H) HC-8 and 7-Me |
| A'3 | 5.52 | 6.25 | 7.26 | 6.12 | 2.00 | 1.67 (3H) HC-7 and HC-8; 0.75 (6H) HC-9 and 8-Me |

5.5 Sugar

The D-mannose unit gives the following $^1$H-NMR signals (delta, ppm) for each of the three factors in $H_2O$:DMSO, 4:1 at pH 4.6, temperature 40 °C, internal standard TMS (delta = 0.00 ppm): 5.40 (one anomeric proton); 4.01, 3.91-3.71, 3.60-3.51 (other protons). The attachment point of the sugar to the peptidic moiety is determined by NMR experiments (Nuclear Overhauser Effect).

5.6 Lactone ring

The presence of a lactone ring is supported by the absorbance at 1760 $cm^{-1}$ in the IR spectrum (see under Example 6, paragraph 6.1). The position of the lactone bond is established by

a) identification of the amino acid contributing to the lactone bond with its carboxylic group

b) identification of the hydroxy-amino acid contributing to the lactone bond with its hydroxyl group.

According to step a), finely ground $CaCl_2$ (2.5 g) is added to a cooled (0-5 °C) solution of $NaBH_4$ (1.5 g) in absolute EtOH (150 ml) with magnetic stirring. After 1.5 hour factor A'2 (1 g) dissolved in dry DMF (60 ml) is added dropwise. The cooled reaction mixture is stirred for 24 hours, then it is cautiously poured into water (600 ml). The pH is adjusted at 5 with aqueous HCl and the solution is desalted on a column filled with a macroporous cross-linked polystyrene resin XAD-2 (Amberlite Rohm and Haas) by eluting first with $H_2O$ to remove salts and after with a mixture 0.01N HCl:$CH_3CN$ 1:1 to recover the reduced peptide. Fractions containing the peptide are combined and concentrated to dryness under vacuum.

The residue is hydrolyzed with 6N HCl at 105 °C for 20 hours. The acidic solution after extraction with ethyl acetate is concentrated under vacuum. The residue is chromatographed on a Dowex 50Wx4 column by eluting with 0.5N HCl. The fractions containing the wanted compound (checked by bidimensional HPTLC: cellulose, first run butanol:acetic acid:$H_2O$ 4:1:5, upper layer, second run pyridine:$H_2O$ 4:1; spots of amino acids are located by spraying with ninhydrin and heating at 120 °C for 5 minutes) are combined and concentrated. The oily residue is purified again by preparative layer chromatography ($SiO_2$, 5 mm thick, butanol:acetic acid:$H_2O$ 4:1:5, upper layer) to obtain the 2-amino-2-(3-chloro-4-hydroxyphenyl)ethyl alcohol: $^1$H-NMR (250 MHz, DMSO-$d_6$) delta, ppm:3.45 (m,CH, partly covered by the water signal), 3.38 (m,$CH_2$), 6.92 (d, CH-5), 7.11 (dd, CH-6, $^3J$ = 8.8 Hz), 7.32 (d, CH-2, $^4J$ = 2.5 Hz). The structure is further confirmed by comparison with an authentic sample prepared from 3-chloro-4-hydroxyphenylglycine (30 mg) which is converted into its methyl ester (MeOH, HCl) then reduced with $Ca(BH_4)_2$ following the procedure reported above. Step b) is accomplished by first reacting factor A'2 with phenylisocyanate. This latter reacts with all the free hydroxyl and amino groups of the peptide giving urethanes and ureas, respectively, that are usually rather resistant to acid hydrolysis carried out, as described in Example 5, paragraph 3. The amount of hydroxyaspartic acid does not decrease, while the amounts of the other hydroxylated amino acids decrease, thus indicating that it is the amino acid involved in the lactone linkage with its hydroxyl group. Accordingly, factor A'2 (100 mg) is dissolved in DMF (2 ml) and phenylisocyanate (300 microliters) is added. The reaction mixture is left at room temperature for 48 hours, quenched by adding $H_2O$ (20 ml) and filtered. The solid compound is hydrolyzed and analyzed for its amino acid composition as described in Example 5, paragraph 3.

The same experiments carried out with factors A'1 and A'3 give corresponding results.

Example 6 - IR, UV and FAB-MS spectra

6.1 The IR spectrum of factor A'2 recorded as nujol mull with a Perkin-Elmer mod. 580 spectrophotometer is shown in Fig. 2 of the accompanying drawings. The following absorption maxima are observed: 3500-3100 (ny NH and ny OH), 3020-2800 (nujol), 1760 (ny C=O lactone), 1630 (ny C=O, amide I), 1510 (delta NH, amide II), 1460 and 1375 (nujol), 1225 (ny C=O, lactone), 1065-980 (ny C=O, sugars), 840 and 815 $cm^{-1}$ (gamma CH aromatic)

The spectra of the other two factors do not show substantial differences.

6.2 The Ultraviolet Spectrum of factor A'2 registered in water with a Perkin Elmer mod. 320 spectrophotometer is given in Fig. 3 of the accompanying drawings. The spectrum exhibits the following absorption maxima: 232 nm (log epsilon 4.64), 270 nm (log epsilon 4.34). The spectra of the other two factors do not show substantial differences.

6.3 The Fast Atom Bombardment Mass Spectra (FAB-MS) are recorded with a MS9/50TC instrument using a thioglycerol/glycerol 1:1 mixture as a matrix. Bombardment gas Xe; kinetic energy 6 keV; accelerating voltage 4kV. The isotopic clusters of the cationized molecular ions $MH^+$ indicate molecular weights of 2375 (factor A'1), 2389 (factor A'2) and 2403 (factor A'3), respectively, (lowest isotope

compositions). These data, and the presence in the spectra of fragment ions corresponding to the loss of 163 units from the respective $MH^+$ ions are in agreement with the structures assigned.

6.4 Elemental analysis

The elemental analysis, after the sample has been previously dried at about 140 °C under inert atmosphere, gives the following approximate percentage composition:

| | factor A'1 | factor A'2 | factor A'3 |
|---|---|---|---|
| C % | 54.2 | 55.2 | 55.8 |
| H % | 5.8 | 5.4 | 6.2 |
| N % | 11.8 | 12.6 | 11.4 |
| Cl % | 3.9 | 4.2 | 4.0 |
| ashes % | 0.9 | 1.3 | 0.7 |

The above values are in agreement with those calculated for the respective di-hydrochloride salts of the three compounds.

**Claims**

**Claims for the following Contracting States : AT, BE, CH, DE, FR, GB, IT, LI, LU, NL, SE,**

**1.** A compound of formula I

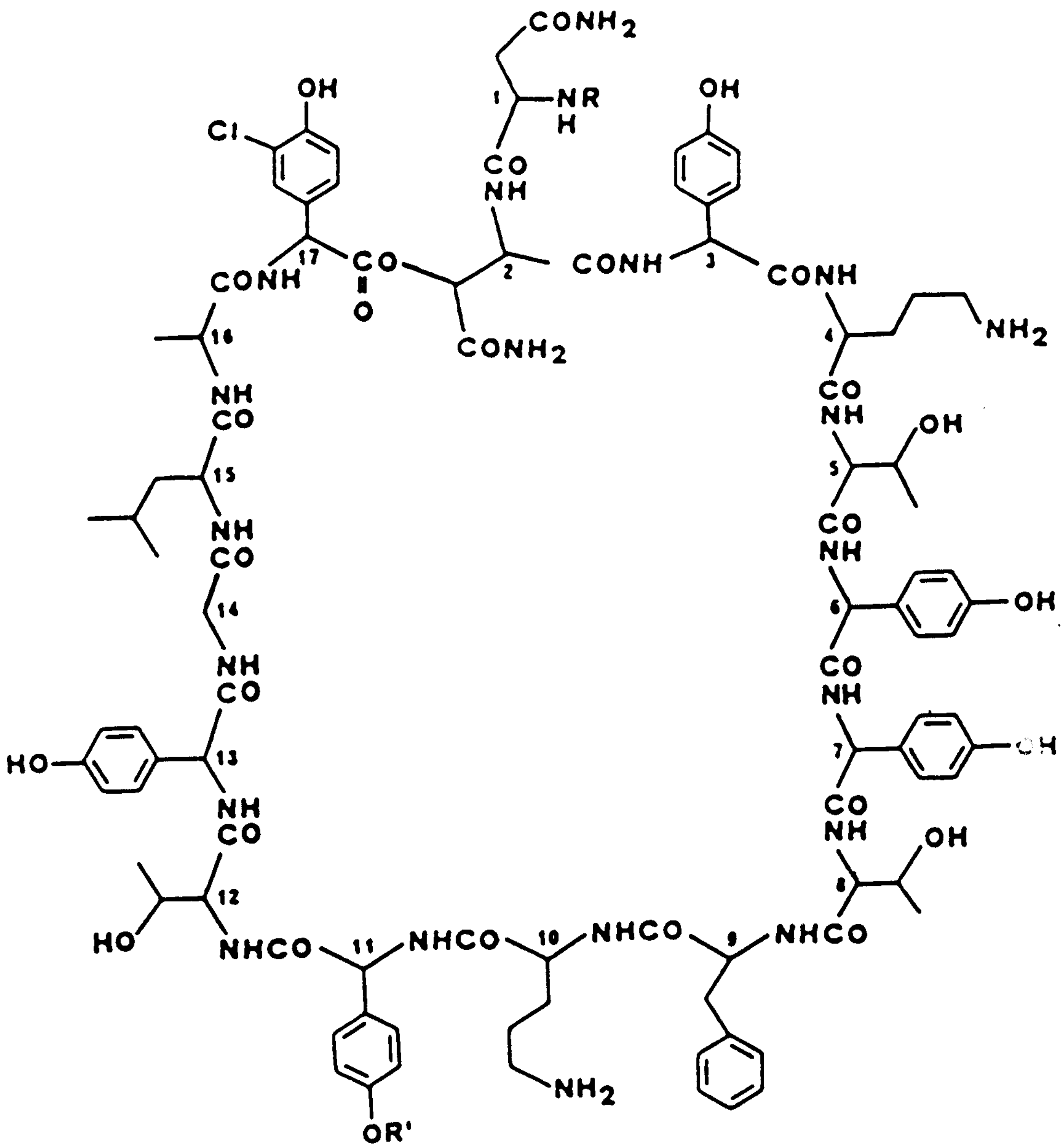

wherein:

R is:

$-CO-CH=CH-CH=CH-CH_2-CH_2-CH_3$,

$-CO-CH=CH-CH=CH-CH_2-CH(CH_3)_2$ or

$-CO-CH=CH-CH=CH-CH_2-CH_2-CH(CH_3)_2$

and R′ is alpha-D-mannopyranosyl and the acid addition salts thereof.

**2.** A compound of claim 1 wherein R is

$-CO-CH=CH-CH=CH-CH_2-CH_2-CH_3$

**3.** A compound of claim 1 wherein R is

$-CO-CH=CH-CH=CH-CH_2-CH(CH_3)_2$

**4.** A compound of claim 1 wherein R is

$-CO-CH=CH-CH=CH-CH_2-CH_2-CH(CH_3)_2$

**5.** An acid addition salt of claim 1 which is the dihydrochloride.

**6.** A process for producing the compounds of claim 1 characterized in that (a) the microorganism Actinoplanes sp. ATCC 33076 or a producing mutant thereof is fermented under submerged aerobic conditions in an aqueous medium containing assimilable sources of carbon and nitrogen and inorganic salts, (b) the mycelium is separated from the fermentation broth, (c) the mycelial mass is extracted with an appropriate solvent system, (d) the crude fermentation product is isolated from said extract and (e) the above antibiotic substances are separated and purified from the isolated crude fermentation product.

**7.** A process as in claim 6 wherein the carbon sources are selected from dextrose, fructose, maltose, sucrose, glycerol and dextrin, the nitrogen sources are selected from soybean meal and malt extract.

**8.** A process as in any of claims 6 and 7 wherein the fermentation is carried out at a temperature between 24 and 35 °C, preferably between 28 and 32 °C, for the period of time during which an increase of antibiotic activity is observed.

**9.** A process as in any of claims 6 through 8 wherein the mycelium is separated from the fermentation broth by filtration or centrifugation at a pH between 4.5 and 5.5, the mycelial mass is extracted with a water miscible organic solvent selected from lower alkanols, acetone and mixtures thereof at a pH between 1.5 and 2.5, and the crude fermentation product is isolated from the extract of the mycelial mass by adding thereto a water miscible organic solvent wherein the acid addition salts of the antibiotic substances are poorly soluble.

**10.** A process as in any of claims 6 through 9 wherein the separation and purification of the antibiotic substances from the isolated crude fermentation product are carried out by preparative HPLC methods.

**11.** A process for producing the compounds of claim 1 characterized in that a substrate containing antibiotic A/16686 factor A1, A2, or A3 or a mixture thereof is contacted with the mycelium of Actinoplanes sp. ATCC 33076 or a natural or artificial mutant thereof capable of producing A/16686 antibiotics.

**12.** A process as in claim 11 wherein the substrate is contacted with the mycelium at a pH around 7 at a temperature between 28 and 35 °C, preferably between 29 and 33 °C, for a period of time varying from 50 to 200 hours.

**13.** A process as in any of claims 11 and 12 wherein the mycelium is previously isolated from the fermentation broth and the contact with the substrate is carried out in a solution of water or a mixture of water with one or more water mixable organic solvents, preferably selected from lower alkanols and acetone.

**14.** A process as in any of claims 11 through 13 wherein the reaction products are separated from the reaction medium by acidifying the mycelial suspension at a pH between 1.5 and 2.5, adding thereto a water mixable organic solvent or a mixture of water mixable organic solvents whenever said solvent(s) is/are not already present in the reaction mixture in amount sufficient for extraction of the reaction product(s) from the mycelial mass, separating the mycelium by centrifugation or filtration and then treating the extract according to any of the claims 9 and 10.

**15.** A compound of claim 1 for use as antibacterial.

**16.** A pharmaceutical composition containing a compound of formula I of claim 1 or a pharmaceutically acceptable acid addition salt thereof.

**17.** Use of a compound of claim 1 for preparing a medicament for combatting infectious diseases due to gram-positive microorganisms.

**18.** A compound of claim 1 for use in the treatment of wound infections or acne.

**19.** A pharmaceutical composition containing a compound of claim 1 as the active ingredient.

**20.** A pharmaceutical composition according to claim 19 particularly useful for the topical treatment of wound infections or acne.

**Claims for the following Contracting States : GR, ES**

**1.** A process for preparing a compound of formula I

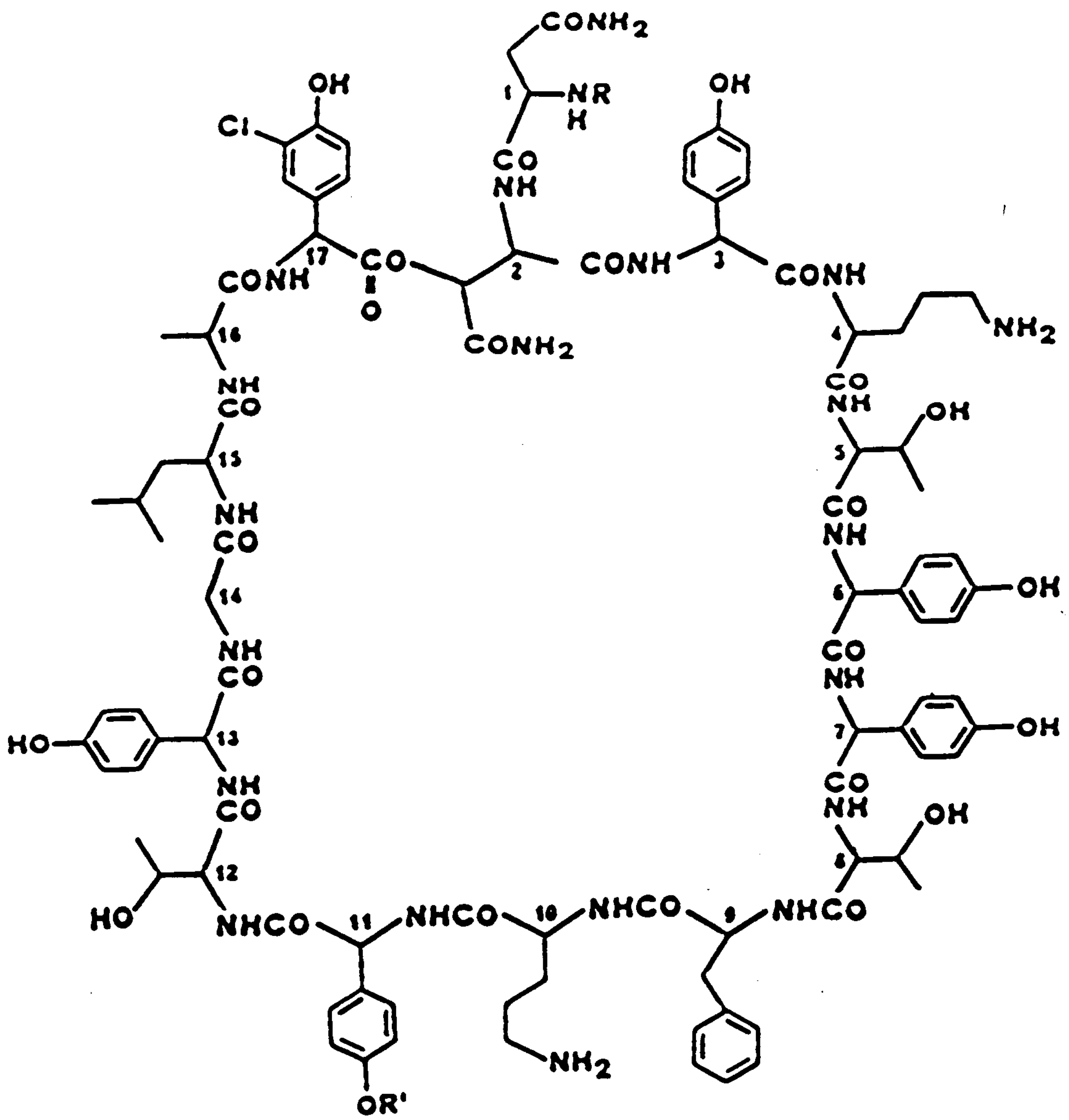

wherein:

R is:

$-CO-CH=CH-CH=CH-CH_2-CH_2-CH_3$,
$-CO-CH=CH-CH=CH-CH_2-CH(CH_3)_2$ or
$-CO-CH=CH-CH=CH-CH_2-CH_2-CH(CH_3)_2$

and R′ is alpha-D-mannopyranosyl and the acid addition salts thereof characterized in that (a) the microorganism Actinoplanes sp. ATCC 33076 or a producing mutant thereof is fermented under submerged aerobic conditions in an aqueous medium containing assimilable sources of carbon and nitrogen and inorganic salts, (b) the mycelium is separated from the fermentation broth, (c) the mycelial mass is extracted with an appropriate solvent system, (d) the crude fermentation product is isolated from said extract and (e) the above antibiotic substances are separated and purified from the isolated crude fermentation product.

**2.** A process as in claim 1 for preparing a compound of formula I wherein R is

$-CO-CH=CH-CH=CH-CH_2-CH_2-CH_3$

**3.** A process as in claim 1 wherein R is

$-CO-CH=CH-CH=CH-CH_2-CH(CH_3)_2$

**4.** A process as in claim 1 wherein R is

$-CO-CH=CH-CH=CH-CH_2-CH_2-CH(CH_3)_2$

**5.** A process as in any of claims 1 through 4 for preparing the compound of claim 1, 2, 3 or 4 in the form of an acid addition salt.

**6.** A process as in claim 5 wherein the acid addition salt is the dihydrochloride.

**7.** A process as in any of the claims 1 through 6 wherein the carbon sources are selected from dextrose, fructose, maltose, sucrose, glycerol and dextrin, the nitrogen sources are selected from soybean meal and malt extract.

**8.** A process as in any of claims 1 through 7 wherein the fermentation is carried out at a temperature between 24 and 35°C, preferably between 28 and 32°C, for the period of time during which an increase of antibiotic activity is observed.

**9.** A process as in any of claims 1 through 8 wherein the mycelium is separated from the fermentation broth by filtration or centrifugation at a pH between 4.5 and 5.5.

**10.** A process as in any of claims 1 through 9 wherein the mycelial mass is extracted with a water miscible organic solvent selected from lower alkanols, acetone and mixtures thereof at a pH between 1.5 and 2.5.

**11.** A process as in any of claims 1 through 10 wherein the crude fermentation product is isolated from the extract of the mycelial mass by adding thereto a water miscible organic solvent wherein the acid addition salts of the antibiotic substances are poorly soluble.

**12.** A process as in any of claims 1 through 11 wherein the separation and purification of the antibiotic substances from the isolated crude fermentation product are carried out by preparative HPLC methods.

**13.** A process as in claim 12 wherein the separation and purification procedures are carried out by employing a C-18 alkyl silanized silicagel column and an eluent mixture of aqueous ammonium formate and acetonitrile.

**14.** A process as in any of claims 1 through 13 wherein during the fermentation process a suitable precursor is added in order to selectively increase the ratio of one of the antibiotic substances of formula I over the others.

**15.** A process as in claim 14 wherein the precursor is selected from leucine, valine, isovaleric acid, isobutyric acid or a salt thereof.

**16.** A process for producing a compound as in claims 1 through 6 characterized in that a substrate containing antibiotic A/16686 factor A1, A2, or A3 or a mixture thereof is contacted with the mycelium of Actinoplanes sp. ATCC 33076 or a natural or artificial mutant thereof capable of producing A/16686 antibiotics.

**17.** A process as in claim 16 wherein the substrate is contacted with the mycelium at a pH around 7 at a temperature between 28 and 35 °C, preferably between 29 and 33 °C, for a period of time varying from 50 to 200 hours.

**18.** A process as in any of claims 16 and 17 wherein the mycelium is previously isolated from the fermentation broth and the contact with the substrate is carried out in a solution of water or a mixture of water with one or more water mixable organic solvents, preferably selected from lower alkanols and acetone.

**19.** A process as in any of claims 16 through 18 wherein the reaction products are separated from the reaction medium by acidifying the mycelial suspension at a pH between 1.5 and 2.5, adding thereto a water mixable organic solvent or a mixture of water mixable organic solvents whenever said solvent(s) is/are not already present in the reaction mixture in amount sufficient for extraction of the reaction product(s) from the mycelial mass, separating the mycelium by centrifugation or filtration and then treating the extract according to any of the claims 11 through 13.

**20.** A process as in claim 16 wherein the contact between the substrate and the mycelium is carried out directly in the fermentation broth, by prolonging the contact of the fermentation product with the mycelium until substantial transformation of A factors to A' factors occurs and the recovery of the reaction products is carried out according to claims 9 through 13.

**Patentansprüche**

**Patentansprüche für folgende Vertragsstaaten : AT, BE, CH, DE, FR, GB, IT, LI, LU, NL, SE**

1. Verbindung der Formel I

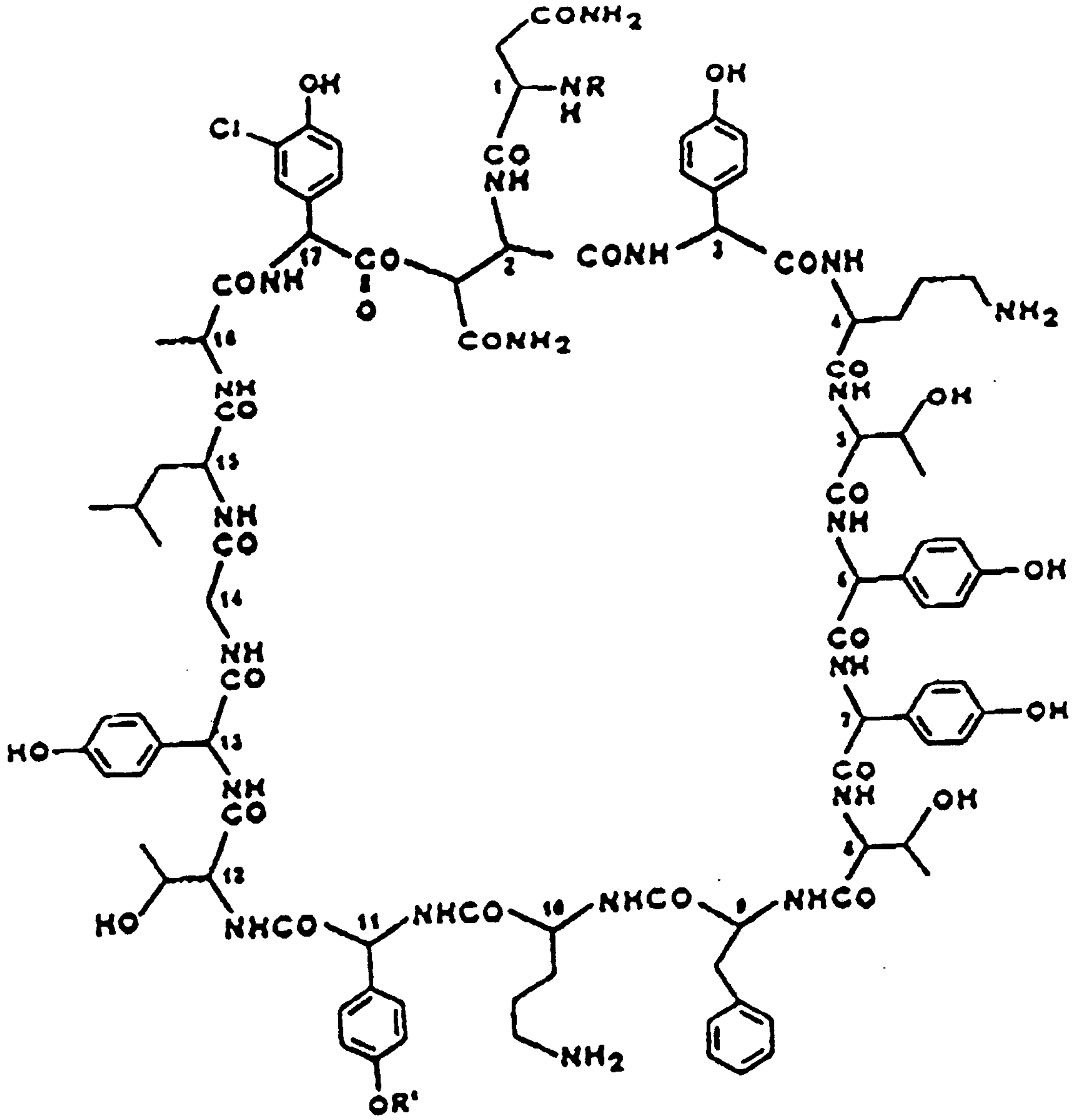

worin

R für

$-CO-CH=CH-CH=CH-CH_2-CH_2-CH_3$,
$-CO-CH=CH-CH=CH-CH_2-CH(CH_3)_2$ oder
$-CO-CH=CH-CH=CH-CH_2-CH_2-CH(CH_3)_2$

steht
und R' α-D-Mannopyranosyl bedeutet, und die Säureadditionsalze davon.

2. Verbindung nach Anspruch 1, dadurch **gekennzeichnet,** daß R für

$-CO-CH=CH-CH=CH-CH_2-CH_2-CH_3$

steht.

3. Verbindung nach Anspruch 1, dadurch **gekennzeichnet,** daß R für

$-CO-CH=CH-CH=CH-CH_2-CH(CH_3)_2$

steht.

4. Verbindung nach Anspruch 1, dadurch **gekennzeichnet,** daß R für

$-CO-CH=CH-CH=CH-CH_2-CH_2-CH(CH_3)_2$

steht.

5. Säureadditionssalz nach Anspruch 1, dadurch **gekennzeichnet,** daß es das Dihydrochlorid ist.

6. Verfahren zur Erzeugung der Verbindungen nach Anspruch 1, dadurch **gekennzeichnet,** daß (a) der Mikroorganismus Actinoplanes sp. ATCC 33076 oder eine produktbildende Mutante davon unter submersen aeroben Bedingungen in einen wäßrigen Medium, das assimilierbare Quellen für Kohlenstoff und Stickstoff und anorganische Salze enthält, fermentiert wird, (b) das Mycel von der Fermentationsbrühe abgetrennt wird, (c) die Mycelmasse mit einem geeigneten Lösungsmittelsystem extrahiert wird, (d) das rohe Fermentationsprodukt aus dem Extrakt isoliert wird, und (e) die vorstehenden antibiotischen Substanzen aus dem isolierten rohen Fermentationsprodukt abgetrennt und gereinigt werden.

7. Verfahren nach Anspruch 6, dadurch **gekennzeichnet,** daß die Kohlenstoffquellen aus Dextrose, Fructose, Maltose, Saccharose, Glycerin und Dextrin, die Stickstoffquellen auf Sojabohnenmehl und Malzextrakt ausgewählt werden.

8. Verfahren nach einem der Ansprüche 6 und 7, dadurch **gekennzeichnet,** daß die Fermentation bei einer Temperatur zwischen 24 und 35°C, bevorzugt zwischen 28 und 32°C, für eine Zeitspanne, währenddessen ein Ansteigen der antibiotischen Aktivität beobachtet wird, durchgeführt wird.

9. Verfahren nach einen der Ansprüche 6 bis 8, dadurch **gekennzeichnet,** daß das Mycel von der Fermentationsbrühe durch Filtration oder Zentrifugation bei einem pH-Wert zwischen 4,5 und 5,5 abgetrennt wird, die Mycelmasse mit einem mit Wasser mischbaren organischen Lösungsmittel, ausgewählt aus niedrigen Alkanolen, Aceton und Gemischen davon, bei einen pH-Wert zwischen 1,5 und 2,5 extrahiert wird und das rohe Fermentationsprodukt aus dem Extrakt der Mycelmasse durch Zusetzen eines mit Wasser mischbaren organischen Lösungsmittels, worin die Säureadditionssalze der antibiotischen Substanzen schlecht löslich sind, isoliert wird.

10. Verfahren nach einem der Ansprüche 6 bis 9, dadurch **gekennzeichnet,** daß die Abtrennung und Reinigung der antibiotischen Substanzen von dem isolierten rohen Fermentationsprodukt mittels präparativer HPLC-Verfahren durchgeführt werden.

11. Verfahren zur Erzeugung der Verbindungen nach Anspruch 1, dadurch **gekennzeichnet,** daß ein Substrat, das den antibiotischen A/16686-Faktor A1, A2 oder A3, oder ein Gemisch davon enthält, mit dem Mycel von Actinoplanes sp. ATCC 33076 oder einer natürlichen oder künstlichen Mutante davon mit der Fähigkeit zur Erzeugung der A/16686-Antibiotika in Kontakt gebracht wird.

12. Verfahren nach Anspruch 11, dadurch **gekennzeichnet,** daß das Substrat mit dem Mycel bei einem pH-Wert um 7 bei einer Temperatur zwischen 28 und 35°C, bevorzugt zwischen 29 und 33°C, für eine Zeitspanne, die von 50 bis 200 Stunden variiert, in Kontakt gebracht wird.

13. Verfahren nach einem der Ansprüche 11 und 12, dadurch **gekennzeichnet,** daß das Mycel zuvor aus der Fermentationsbrühe isoliert wird und der Kontakt mit den Substrat in einer Lösung aus Wasser oder einem Gemisch aus Wasser mit einem oder mehreren mit Wasser mischbaren organischen Lösungsmitteln, bevorzugt ausgewählt aus niedrigen Alkanolen und Aceton, durchgeführt wird.

14. Verfahren nach einem der Ansprüche 11 bis 13, dadurch **gekennzeichnet,** daß die Reaktionsprodukte von dem Reaktionsmedium durch Ansäuern der Mycelsuspension bei einem pH-Wert zwischen 1,5 und

2,5, Zusatz eines mit Wasser mischbaren organischen Lösungsmittels oder eines Gemisches aus mit Wasser mischbaren organischen Lösungsmitteln, sofern das Lösungsmittel bzw. die Lösungsmittel noch nicht in dem Reaktionsgemisch in einer Menge, die zur Extraktion des Reaktionsproduktes bzw. der Reaktionsprodukte von der Mycelmasse ausreichend ist, vorhanden ist bzw. sind, dazu, Abtrennen des Mycels durch Zentrifugation oder Filtration und anschließende Behandlung des Extrakts nach einem der Ansprüche 9 und 10, abgetrennt werden.

**15.** Verbindung nach Anspruch 1 zur Verwendung als antibakterielles Mittel.

**16.** Pharmazeutisches Präparat, enthaltend eine Verbindung der Formel I nach Anspruch 1 oder ein pharmazeutisch annehmbares Säureadditionssalz davon.

**17.** Verwendung einer Verbindung nach Anspruch 1 zur Herstellung eines Medikaments zur Bekämpfung von infektiösen auf gram-positive Mikroorganismen zurückzuführende Erkrankungen.

**18.** Verbindung nach Anspruch 1 zur Verwendung bei der Behandlung von Wundinfektionen oder Akne.

**19.** Pharmazeutisches Präparat, enthaltend eine Verbindung nach Anspruch 1 als Wirkstoff.

**20.** Pharmazeutisches Präparat nach Anspruch 19, insbesondere zur Verwendung zur topischen Behandlung von Wundinfektionen oder Akne.

**Patentansprüche für folgende Vertragsstaaten : GR, ES**

1. Verfahren zur Herstellung einer Verbindung der Formel I

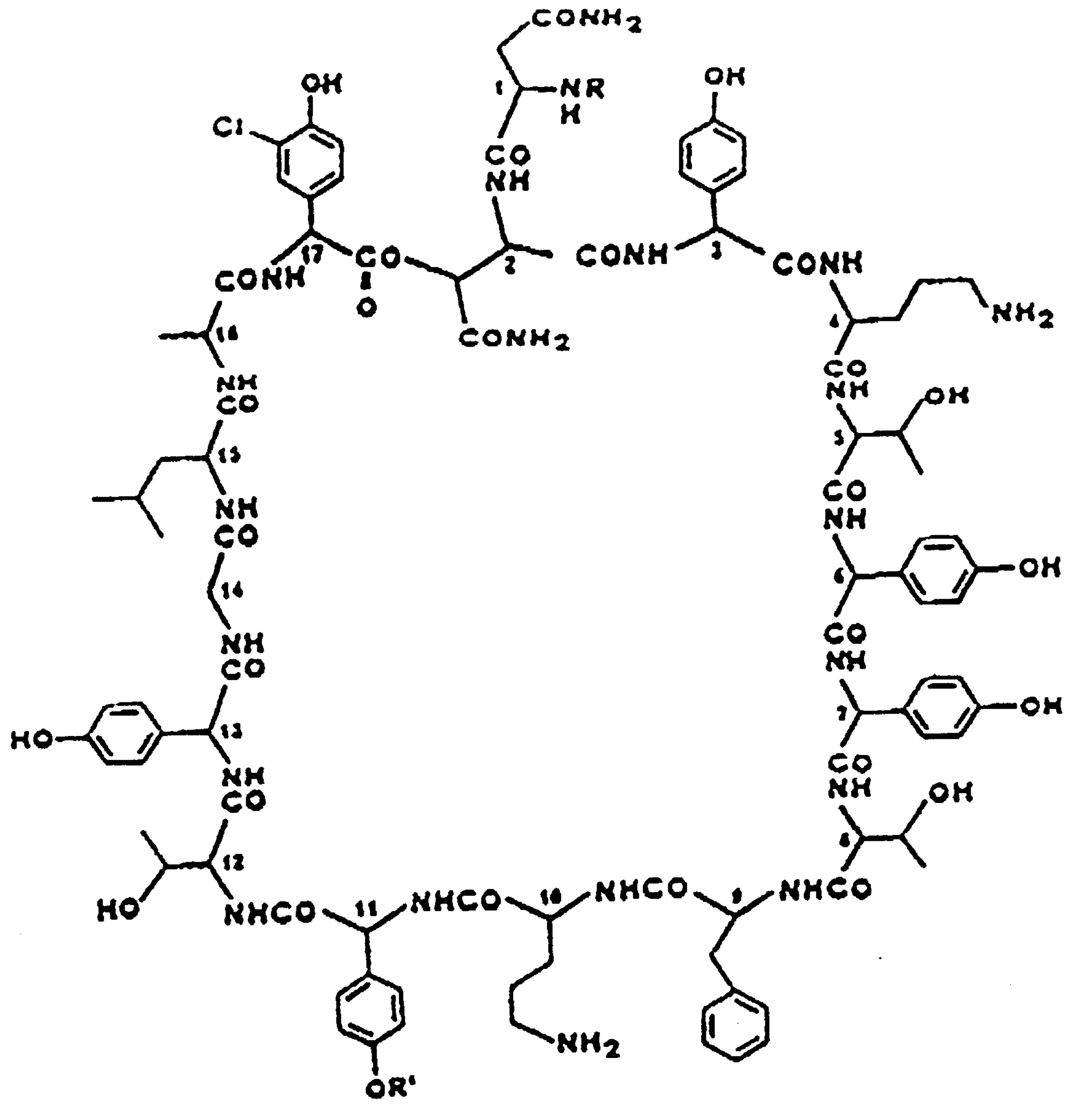

worin

R für $-CO-CH=CH-CH=CH-CH_2-CH_2-CH_3$, $-CO-CH=CH-CH=CH-CH_2-CH(CH_3)_2$ oder $-CO-CH=CH-CH=CH-CH_2-CH_2-CH(CH_3)_2$

steht,

und R' α-D-mannopyranosyl bedeutet und der SäureaddiMtionssalze davon, dadurch **gekennzeichnet,** daß (a) der Mikroorganismus Actinoplanes sp. ATCC 33076 oder eine produktbildende Mutante davon unter submersen aeroben Bedingungen in einem wäßrigen Medium, das assimilierbare Quellen für Kohlenstoff und Stickstoff und anorganische Salze enthält, fermentiert wird, (b) das Mycel von der Fermentationsbrühe abgetrennt wird, (c) die Mycelmasse mit einem geeigneten Lösungsmittelsystem extrahiert wird, (d) das rohe Fermentationsprodukt von dem Extrakt isoliert wird, und (e) die vorstehenden antibiotischen Substanzen aus dem isolierten rohen Fermentationsprodukt abgetrennt und gereinigt werden.

2. Verfahren nach Anspruch 1 zur Herstellung einer Verbindung der Formel I, worin R für

$-CO-CH=CH-CH=CH-CH_2-CH_2-CH_3$

steht.

3. Verfahren nach Anspruch 1, dadurch **gekennzeichnet,** daß R für

$-CO-CH=CH-CH=CH-CH_2-CH(CH_3)_2$

steht.

4. Verfahren nach Anspruch 1, dadurch **gekennzeichnet,** daß R für

$-CO-CH=CH-CH=CH-CH_2-CH_2-CH(CH_3)_2$

steht.

5. Verfahren nach einem der Ansprüche 1 bis 4 zur Herstellung der Verbindung der Ansprüche 1, 2, 3 oder 4 in Form eines Säureadditionssalzes.

6. Verfahren nach Anspruch 5, dadurch **gekennzeichnet,** daß das Säureadditionssalz das Dihydrochlorid ist.

7. Verfahren nach einem der Ansprüche 1 bis 6, dadurch **gekennzeichnet,** daß die Kohlenstoffquellen aus Dextrose, Fructose, Maltose, Saccharose, Glycerin und Dextrin ausgewählt werden, die Stickstoffquellen aus Sojabohnenmehl und Malzextrakt ausgewählt werden.

8. Verfahren nach einem der Ansprüche 1 bis 7, dadurch **gekennzeichnet,** daß die Fermentation bei einer Temperatur zwischen 24 und 35 °C, bevorzugt zwischen 28 und 32 °C, für eine Zeitspanne, während der ein Ansteigen der antibiotischen Aktivität beobachtet wird, durchgeführt wird.

9. Verfahren nach einem der Ansprüche 1 bis 8, dadurch **gekennzeichnet,** daß das Mycel von der Fermentationsbrühe durch Filtration oder Zentrifugation bei einer pH-Wert zwischen 4,5 und 5,5 abgetrennt wird.

10. Verfahren nach einem der Ansprüche 1 bis 9, dadurch **gekennzeichnet,** daß die Mycelmasse mit einen mit Wasser mischbaren organischen Lösungsmittel, ausgewählt aus niedrigen Alkanolen, Aceton und Gemischen davon, bei einem pH-Wert zwischen 1,5 und 2,5 extrahiert wird.

11. Verfahren nach einem der Ansprüche 1 bis 10, dadurch **gekennzeichnet,** daß das rohe Fermentationsprodukt aus dem Extrakt der Mycelmasse durch Zugabe eines mit Wasser mischbaren organischen Lösungsmittels, worin die Säureadditionssalze der antibiotischen Substanzen schlecht löslich sind, dazu, isoliert wird.

12. Verfahren nach einem der Ansprüche 1 bis 11, dadurch **gekennzeichnet,** daß die Abtrennung und Reinigung der antibiotischen Substanzen aus dem isolierten rohen Fermentationsprodukt durch präparative HPLC-Verfahren durchgeführt werden.

13. Vorfahren nach Anspruch 12, dadurch **gekennzeichnet,** daß die Abtrennungs- und Reinigungsverfahren unter Verwendung einer C-18-alkylsilanisierten Silicagelsäure und eines Elutionsgemisches aus wäßrigem Ammoniumformiat und Acetonitril durchgeführt werden.

14. Verfahren nach einem der Ansprüche 1 bis 13, dadurch **gekennzeichnet,** daß während des Fermentationsprozesses ein geeigneter Vorläufer zugesetzt wird, um selektiv das Verhältnis einer der antibiotischen Substanzen der Formel I gegenüber den anderen zu erhöhen.

**15.** Verfahren nach Anspruch 14, dadurch **gekennzeichnet,** daß der Vorläufer aus Leucin, Valin, Isovaleriansäure, Isobuttersäure oder einem Salz davon ausgewählt wird.

**16.** Verfahren zur Erzeugung einer Verbindung nach den Ansprüchen 1 bis 6, dadurch **gekennzeichnet,** daß ein Substrat, das den antibiotischen A/16686-Faktor A1, A2 oder A3 oder ein Gemisch davon enthält, mit dem Mycel von Actinoplanes sp. ATCC 33076 oder einer natürlichen oder künstlichen Mutante davon mit der Fähigkeit zur Erzeugung der A/16686-Antibiotika in Kontakt gebracht wird.

**17.** Verfahren nach Anspruch 16, dadurch **gekennzeichnet,** daß das Substrat mit dem Mycel bei einem pH-wert in Bereich von 7 bei einer Temperatur zwischen 28 und 35 ° C, bevorzugt zwischen 29 und 33 ° C, für eine Zeitspanne, die von 50 bis 200 Stunden variiert, in Kontakt gebracht wird.

**18.** Verfahren nach einem der Ansprüche 16 und 17, dadurch **gekennzeichnet,** daß das Mycel zuvor aus der Fermentationsbrühe isoliert wird und daß der Kontakt mit dem Substrat in einer Lösung aus Wasser oder einem Gemisch aus Wasser mit einem oder mehreren mit Wasser mischbaren organischen Lösungsmitteln, bevorzugt ausgewählt aus niedrigen Alkanolen und Aceton, durchgeführt wird.

**19.** Verfahren nach einem der Ansprüche 16 bin 18, dadurch **gekennzeichnet,** daß die Reaktionsprodukte von dem Reaktionsmedium durch Ansäuern der Mycelsuspension bei einem pH-Wert zwischen 1,5 und 2,5, Zugabe eines mit Wasser mischbaren organischen Lösungsmittels oder eines Gemisches aus mit Wasser mischbaren organischen Lösungsmitteln, sofern das bzw. die Lösungsmittel noch nicht in den Reaktionsgemisch in einer Menge, die zur Extraktion des Reaktionsproduktes bzw. der Reaktionsprodukte aus der Mycelmasse ausreicht, vorhanden ist bzw. sind, dazu, Abtrennen des Mycels durch Zentrifugation oder Filtration und anschließende Behandlung des Extrakts nach einem der Ansprüche 11 bis 13, abgetrennt werden.

**20.** Verfahren nach Anspruch 16, dadurch **gekennzeichnet,** daß der Kontakt zwischen dem Substrat und den Mycel direkt in der Fermentationsbrühe durchgeführt wird, indem der Kontakt des Fermentationsprodukts mit den Mycel verlängert wird, bis eine wesentliche Umwandlung der A-Faktoren in A'-Faktoren eintritt und die Gewinnung der Reaktionsprodukte nach den Ansprüchen 9 bis 13 durchgeführt wird.

**Revendications**

**Revendications pour les Etats contractants suivants : AT, BE, CH, DE, FR, GB, IT, LI, LU, NL, SE,**

1. Composé de formule I

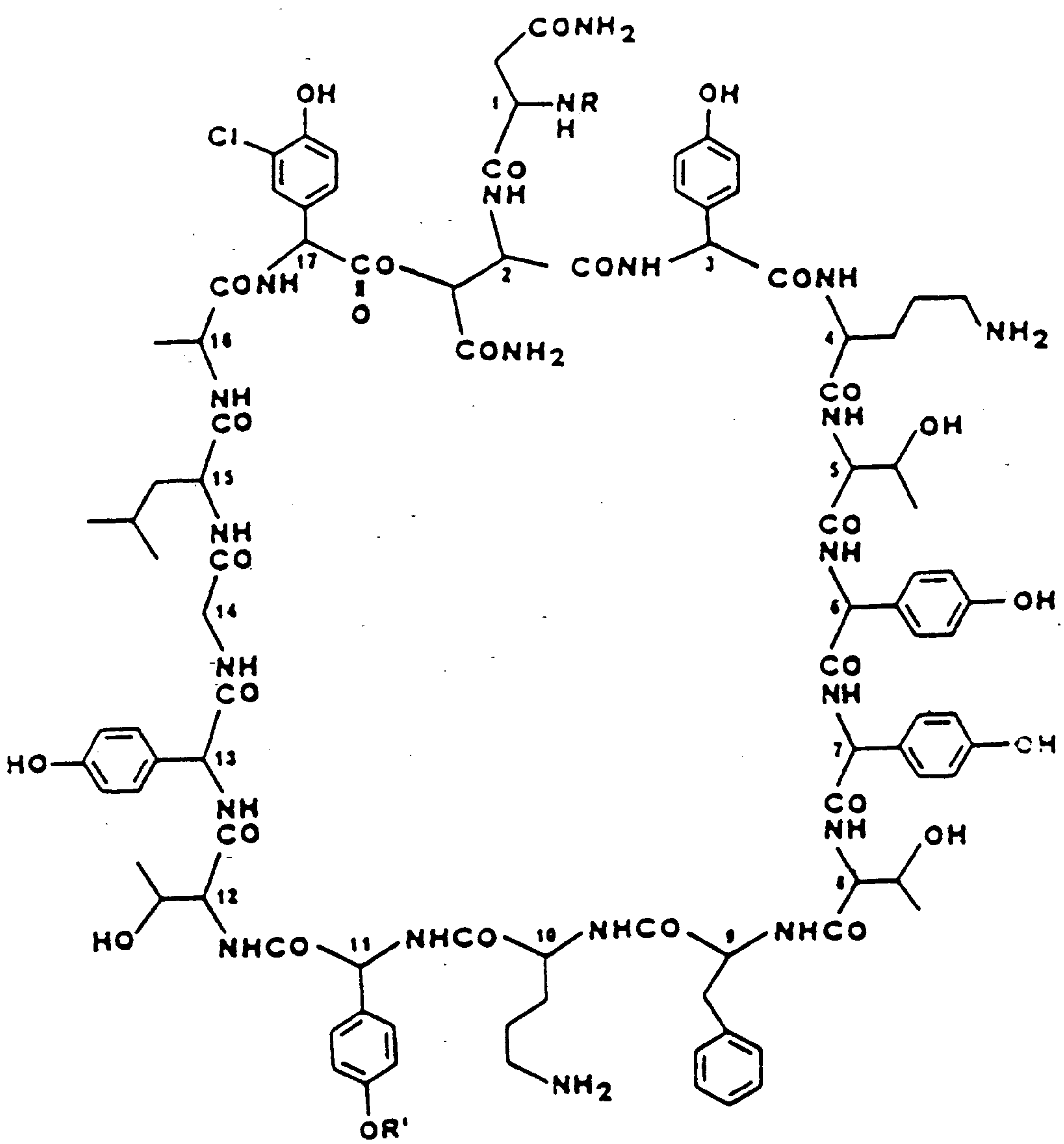

dans lequel

R est :

$-CO-CH=CH-CH=CH-CH_2-CH_2-CH_3$,

$-CO-CH=CH-CH=CH-CH_2-CH(CH_3)_2$ ou

$-CO-CH=CH-CH=CH-CH_2-CH_2-CH(CH_3)_2$

et R' est l'alpha-D-mannopyranosyle et les sels d'addition aux acides de celui-ci.

2. Composé selon la revendication 1 dans lequel R est

$-CO-CH=CH-CH=CH-CH_2-CH_2-CH_3$.

3. Composé selon la revendication 1 dans lequel R est

$-CO-CH=CH-CH=CH-CH_2-CH(CH_3)_2$.

4. Composé selon la revendication 1 dans lequel R est

$-CO-CH=CH-CH=CH-CH_2-CH_2-CH(CH_3)_2$.

5. Sel d'addition aux acides selon la revendication 1 qui est le dichlorhydrate.

6. Procédé pour la production des composés selon la revendication 1, caractérisé en ce que (a) on fait fermenter le micro-organisme Actinoplanes ATCC 33076 ou un mutant producteur de celui-ci dans des conditions aérobies immergées dans un milieu aqueux contenant des sources assimilables de carbone et d'azote et des sels minéraux, (b) on sépare le mycélium du bouillon de fermentation, (c) on extrait la masse mycélienne avec un système de solvants approprié, (d) on isole le produit de fermentation brut dudit extrait et (e) on sépare et on purifie les substances antibiotiques précitées à partir du produit de fermentation brut isolé.

7. Procédé selon la revendication 6, dans lequel les sources de carbone sont sélectionnées parmi le dextrose, le fructose, le maltose, le saccharose, le glycérol et la dextrine, et les sources d'azote sont sélectionnées parmi la farine de soja et l'extrait de malt.

8. Procédé selon l'une quelconque des revendications 6 et 7, dans lequel la fermentation est effectuée à une température comprise entre 24 et 35 °C, de préférence entre 28 et 32 °C pendant la période durant laquelle une augmentation de l'activité antibiotique est observée.

9. Procédé selon l'une quelconque des revendications 6 à 8, dans lequel le mycélium est séparé du bouillon de fermentation par filtration ou centrifugation à un pH compris entre 4,5 et 5,5, la masse mycélienne est extraite avec un solvant organique miscible à l'eau sélectionné parmi les alcanols inférieurs, l'acétone et les mélanges de ceux-ci à un pH compris entre 1,5 et 2,5, et le produit de fermentation brut est isolé à partir de l'extrait de la masse mycélienne par addition à celui d'un solvant organique miscible à l'eau dans lequel les sels d'addition aux acides des substances antibiotiques sont faiblement solubles.

10. Procédé selon l'une quelconque des revendications 6 à 9, dans lequel la séparation et la purification des substances antibiotiques à partir du produit de fermentation brut isolé sont effectuées par des méthodes d'HPLC préparative.

11. Procédé pour produire les composés selon la revendication 1, caractérisé en ce qu'un substrat contenant l'antibiotique A/16686 facteur A1, A2 ou A3 ou un mélange de ceux-ci, est mis en contact avec le mycélium d'Actinoplanes ATCC 33076 ou un mutant naturel ou artificiel de celui-ci capable de produire les antibiotiques A/16686.

12. Procédé selon la revendication 11, dans lequel le substrat est mis en contact avec le mycélium à un pH d'environ 7 à une température comprise entre 28 et 35 °C, de préférence entre 29 et 33 °C, pendant une durée variant de 50 à 200 heures.

13. Procédé selon l'une quelconque des revendications 11 et 12, dans lequel le mycélium est préalablement isolé du bouillon de fermentation et la mise en contact avec le substrat est effectuée dans une solution d'eau ou d'un mélange d'eau avec un ou plusieurs solvants organiques miscibles à l'eau, de préférence sélectionné(s) parmi les alcanols inférieurs et l'acétone.

14. Procédé selon l'une quelconque des revendications 11 à 13, dans lequel les produits de réaction sont séparés du milieu réactionnel par acidification de la suspension mycélienne à un pH compris entre 1,5 et 2,5, par addition à celle-ci d'un solvant organique miscible à l'eau ou d'un mélange de solvants organiques miscibles à l'eau, chaque fois que le(s)dit(s) solvant(s) n'est/ne sont pas déjà présent(s) dans le mélange réactionnel en une quantité suffisante pour l'extraction du/des produit(s) de réaction à partir de la masse mycélienne, par séparation du mycélium par centrifugation ou filtration puis

traitement de l'extrait selon l'une quelconque des revendications 9 et 10.

**15.** Composé selon la revendication 1 pour son utilisation comme bactéricide.

**16.** Composition pharmaceutique contenant un composé de formule I selon la revendication 1 ou un sel d'addition avec un acide phamaceutiquement acceptable de celui-ci.

**17.** Utilisation d'un composé selon la revendication 1 pour la préparation d'un médicament pour combattre des maladies infectieuses dues à des micro-organismes grampositifs.

**18.** Composé selon la revendication 1 pour son utilisation dans le traitement d'infections de plaies ou d'acné.

**19.** Composition pharmaceutique contenant un composé selon la revendication 1 comme constituant actif.

**20.** Composition pharmaceutique selon la revendication 19 utile en particulier pour le traitement topique d'infections de plaies ou d'acné.

**Revendications pour les Etats contractants suivants : GR, ES**

1. Procédé pour la préparation d'un composé de formule I:

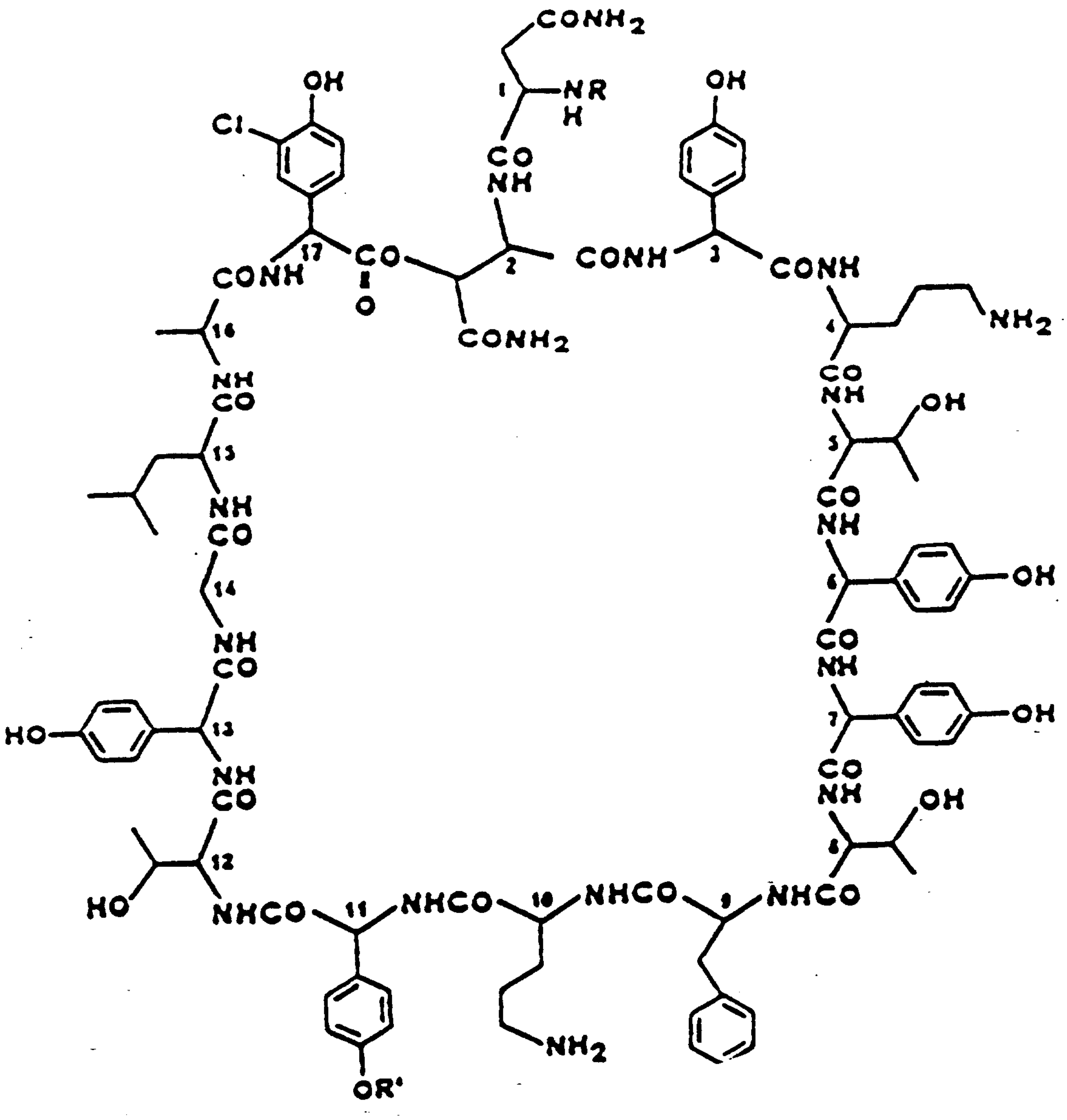

dans lequel :

R est :

$-CO-CH=CH-CH=CH-CH_2-CH_2-CH_3$,
$-CO-CH=CH-CH=CH-CH_2-CH(CH_3)_2$ ou
$-CO-CH=CH-CH=CH-CH_2-CH_2-CH(CH_3)_2$

et R' est l'alpha-D-mannopyranosyle et les sels d'addition aux acides de celui-ci, caractérisé en ce que (a) on fait fermenter le micro-organisme Actinoplanes ATCC 33076 ou un mutant producteur de celui-ci dans des conditions aérobies immergées dans un milieu aqueux contenant des sources assimilables de carbone et d'azote et des sels minéraux, (b) on sépare le mycélium du bouillon de fermentation, (c) on extrait la masse mycélienne avec un système de solvants approprié, (d) on isole le produit de fermentation brut dudit extrait et (e) on sépare et on purifie les substances antibiotiques précitées à partir du produit de fermentation brut isolé.

2. Procédé selon la revendication 1 pour la préparation d'un composé de formule I dans lequel R est

$-CO-CH=CH-CH=CH-CH_2-CH_2-CH_3$.

3. Procédé selon la revendication 1, dans lequel R est

$-CO-CH=CH-CH=CH-CH_2-CH(CH_3)_2$.

4. Procédé selon la revendication 1, dans lequel R est

$-CO-CH=CH-CH=CH-CH_2-CH_2-CH(CH_3)_2$.

5. Procédé selon l'une quelconque des revendications 1 à 4 pour la préparation du composé de la revendication 1, 2, 3 ou 4 sous la forme d'un sel d'addition avec un acide.

6. Procédé selon la revendication 5, dans lequel le sel d'addition avec un acide est le dichlorhydrate.

7. Procédé selon l'une quelconque des revendications 1 à 6, dans lequel les sources de carbone sont sélectionnées parmi le dextrose, le fructose, le maltose, le saccharose, le glycérol et la dextrine, et les sources d'azote sont sélectionnées parmi la farine de soja et l'extrait de malt.

8. Procédé selon l'une quelconque des revendications 1 à 7, dans lequel la fermentation est effectuée à une température comprise entre 24 et 35 ° C, de préférence entre 28 et 32 ° C pendant la période durant laquelle une augmentation de l'activité antibiotique est observée.

9. Procédé selon l'une quelconque des revendications 1 à 8, dans lequel le mycélium est séparé du bouillon de fermentation par filtration ou centrifugation à un pH compris entre 4,5 et 5,5.

10. Procédé selon l'une quelconque des revendications 1 à 9, dans lequel la masse mycélienne est extraite avec un solvant organique miscible à l'eau sélectionné parmi les alcanols inférieurs, l'acétone et les mélanges de ceux-ci à un pH compris entre 1,5 et 2,5.

11. Procédé selon l'une quelconque des revendications 1 à 10, dans lequel le produit de fermentation brut est isolé de l'extrait de la masse mycélienne par addition à celui-ci d'un solvant organique miscible à l'eau dans lequel les sels d'addition aux acides des substances antibiotiques sont faiblement solubles.

12. Procédé selon l'une quelconque des revendications 1 à 11, dans lequel la séparation et la purification des substances antibiotiques à partir du produit de fermentation brut isolé sont effectuées par des méthodes d'HPLC préparative.

13. Procédé selon la revendication 12, dans lequel les méthodes de séparation et de purification sont appliquées en employant une colonne de gel de silice silanisé avec des groupes alkyles en $C_{18}$ et un mélange éluant de formiate d'ammonium aqueux et d'acétonitrile.

14. Procédé selon l'une quelconque des revendications 1 à 13, dans lequel pendant le processus de fermentation, un précurseur convenable est additionné afin d'augmenter sélectivement le rapport d'une des substances antibiotiques de formule I relativement aux autres.

15. Procédé selon la revendication 14, dans lequel le précurseur est sélectionné parmi la leucine, la valine, l'acide isovalérique, l'acide isobutyrique ou un sel de ceux-ci.

16. Procédé pour la production d'un composé selon la revendication 1 à 6, caractérisé en ce qu'un substrat contenant l'antibiotique A/16686 facteur A1, A2 ou A3 ou un mélange de ceux-ci, est mis en contact avec le mycélium d'Actinoplanes ATCC 33076 ou un mutant naturel ou artificiel de celui-ci capable de produire les antibiotiques A/16686.

17. Procédé selon la revendication 16, dans lequel le substrat est mis en contact avec le mycélium à un pH d'environ 7 à une température comprise entre 28 et 35 ° C, de préférence entre 29 et 33 ° C,

pendant une durée variant de 50 à 200 heures.

18. Procédé selon l'une quelconque des revendications 16 et 17, dans lequel le mycélium est préalablement isolé du bouillon de fermentation et la mise en contact avec le substrat est effectuée dans une solution d'eau ou d'un mélange d'eau avec un ou plusieurs solvants organiques miscibles à l'eau, de préférence sélectionné(s) parmi les alcanols inférieurs et l'acétone.

19. Procédé selon l'une quelconque des revendications 16 à 18, dans lequel les produits de réaction sont séparés du milieu réactionnel par acidification de la suspension mycélienne à un pH compris entre 1,5 et 2,5, par addition à celle-ci d'un solvant organique miscible à l'eau ou d'un mélange de solvants organiques miscibles à l'eau, chaque fois que le(s)dit(s) solvant(s) n'est/ne sont pas déjà présent(s) dans le mélange réactionnel en une quantité suffisante pour l'extraction du/des produit(s) de réaction à partir de la masse mycélienne, par séparation du mycélium par centrifugation ou filtration puis traitement de l'extrait selon l'une quelconque des revendications 11 à 13.

20. Procédé selon la revendication 16, dans lequel le contact entre le substrat et le mycélium est effectué directement dans le bouillon de fermentation, en prolongeant le contact du produit de fermentation avec le mycélium jusqu'à ce qu'une transformation substantielle des facteurs A en facteurs A' se produise et la récupération des produits de réaction est effectuée selon les revendications 9 à 13.

$^1H$-NMR Spectrum of Factor A'2

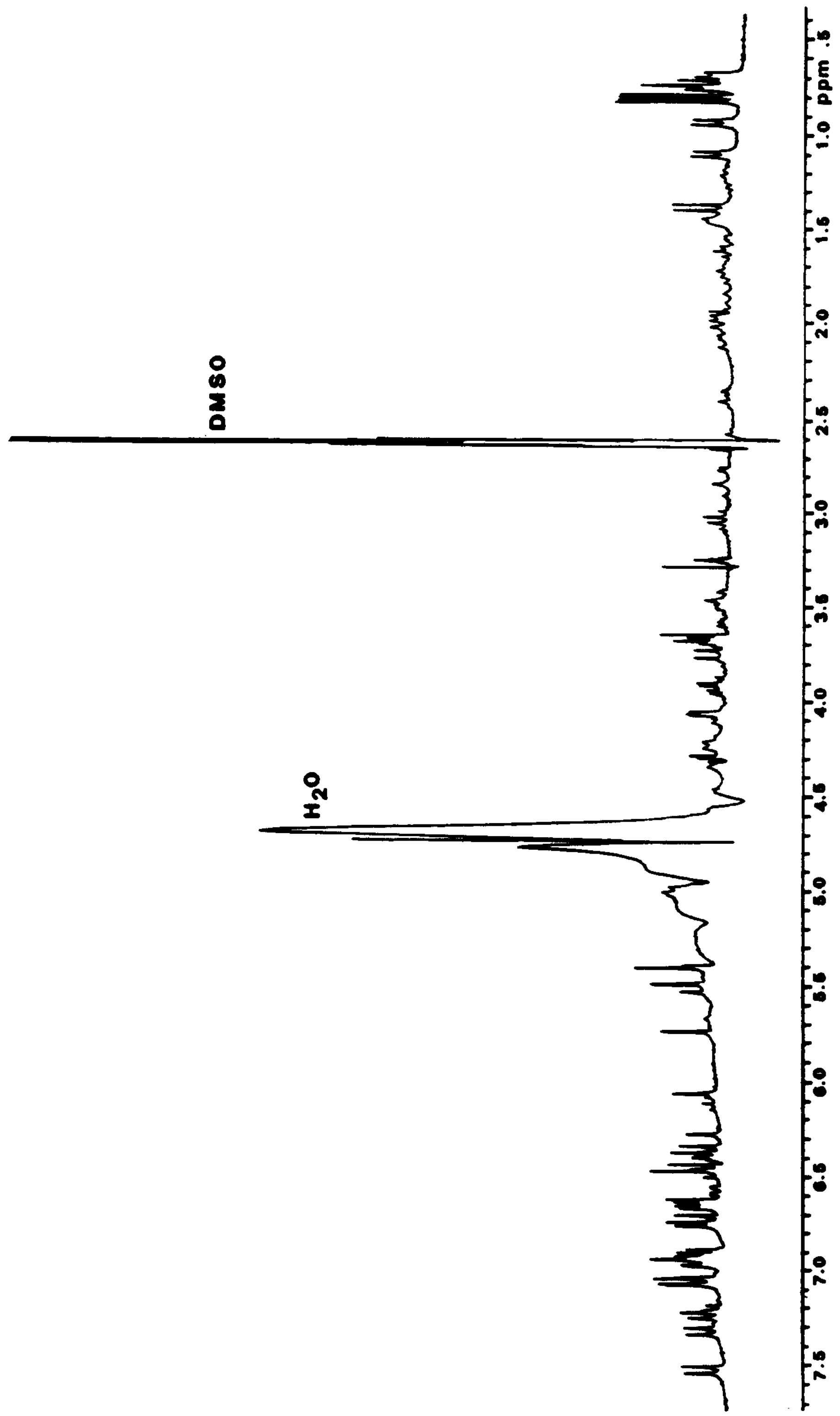


Fig. 1

IR SPECTRUM OF FACTOR A'2

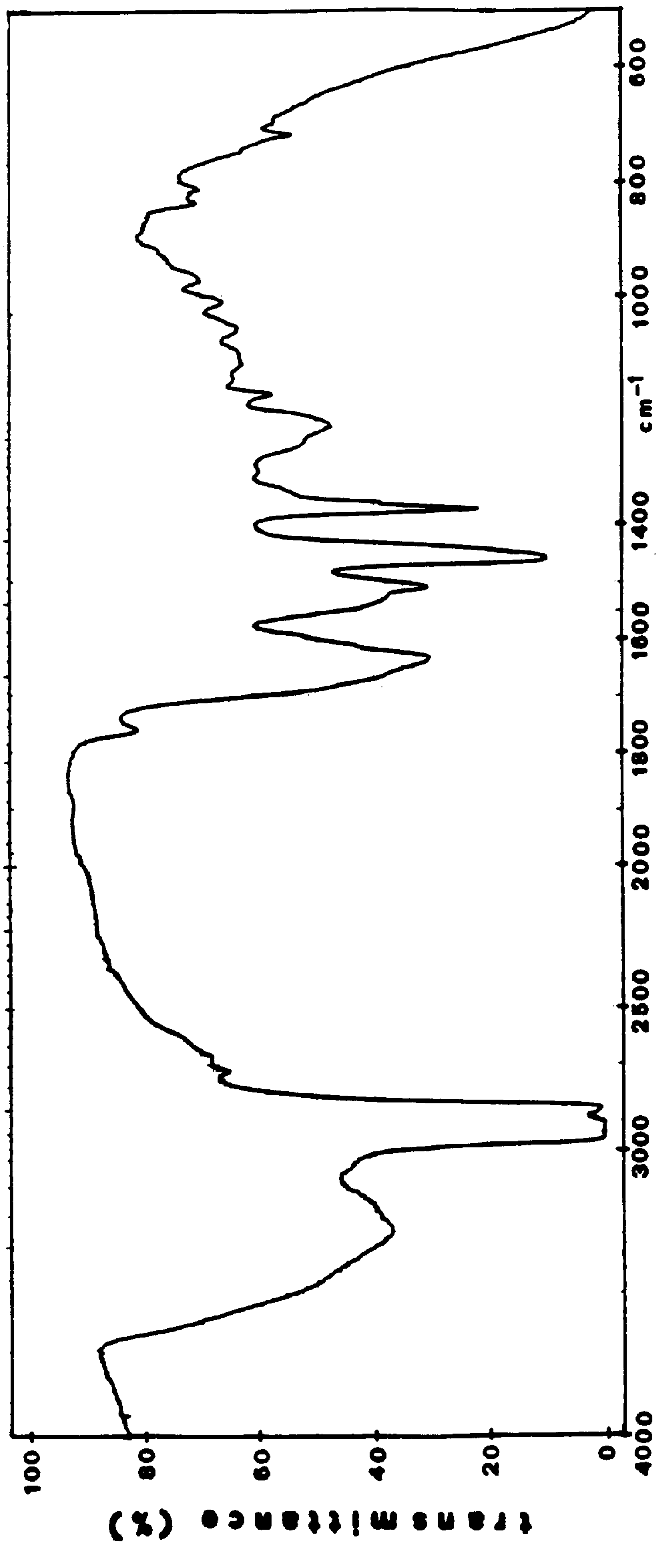


FIG. 2

Ultraviolet Spectrum of Factor A'2

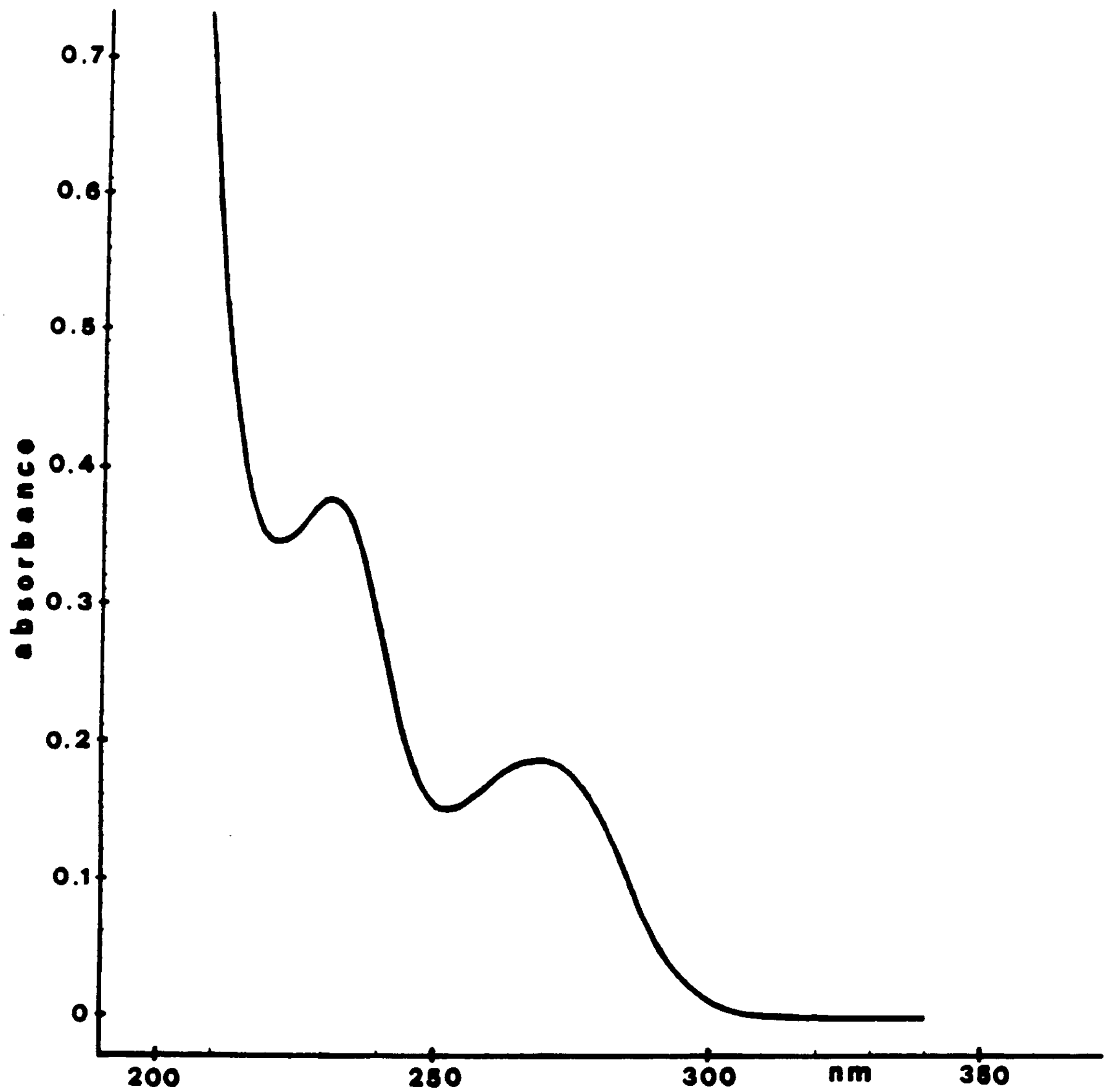


Fig. 3